# EUROPEAN PATENT APPLICATION

(11) **EP 4 595 982 A1**
(43) Date of publication of application: **06.08.2025**
(21) Application number: 24305195.0
(22) Date of filing: 05.02.2024
(51) Int. Cl.: A61K 47/68, C07K 1/113

(54) **MONOCONJUGATES AND THEIR PROCESS OF PREPARATION**

(71) Applicant: Syndivia, 67083 Strasbourg (FR)
(72) Inventor: SCHLIMPEN, Fabian, 67100 STRASBOURG (FR); DATTLER, Damien, 67000 STRASBOURG (FR); SEMENCHENKO, Constantin, 67400 ILLKIRCH-GRAFFENSTADEN (FR); KOLODYCH, Sergii, 67200 STRASBOURG (FR); KONIEV, Oleksandr, 67200 STRASBOURG (FR)
(74) Representative: Lavoix

(57) **Abstract**

The present application is directed to novel rebridged conjugates having a degree of conjugation of 1 as key intermediates towards antibody-drug conjugates and the process leading to the same.

## Description

Modern cancer therapeutics aim at increasing specificity for cancer cells thus reducing toxicity for healthy tissues.

Antibody drug conjugates (ADCs) represent a promising class with this potential exploiting their antigen-specificity for cancer-related targets to deliver cytotoxic compounds to cancer cells.

ADCs are highly valuable tools in cancer treatment, as they enable the targeted delivery of potent cytotoxins to cancer cells. However, the current methods used to create ADCs still have various limitations, particularly in terms of site-selectivity during payload conjugation and controlling the drug-to-antibody ratio (DAR), i.e., the number of drugs per antibody. Considering the accelerated clearance and reduced safety profile witnessed in first generation ADCs featuring drug-to-antibody ratios (DARs) frequently reaching eight, a shift in the prevailing paradigm has occurred in favour of conjugates with lower DAR values. Particularly challenging is the generation of ADCs with one single payload per antibody (DAR=1).

EP3424538 suggests a conjugation method that controls the degree of conjugation and leads to ADCs mixtures where said ADCs have the same DAR, including 1 and may vary by the site of conjugation.

Recent endeavours have also pivoted towards disulfide re-bridging reagents specifically reacting with cysteine-thiols from (partially) reduced antibodies : they contain two cysteine-reactive groups that may undergo reaction with reduced interchain disulfides in an antibody to effect covalent, thus rebridging of the antibody chains in a site-selective fashion. These re-bridging agents also furnish supplementary functional moieties to enable subsequent payload-linker conjugation.

Various re-bridging strategies are under investigation. They should ensure the covalent linkage of the heavy and light chains of the antibodies. Importantly, it is desirable to provide strategies allowing drug conjugation with the controlled conjugation leading to one payload per antibody. In fact, the formation of homogeneous ADCs with DAR = 1 remains particularly challenging.

In mAbs vol.11 (3), 2019, White et al disclose a re-bridging strategy to prepare homogenous ADCs with DAR of one using an engineered antibody and a PBD dimer. However, the proposed strategy requires an engineered antibody and the use of a long linker, which can potentially impact the PK/PD parameters of the ADC.

There is thus still a need to provide more versatile methods, which may apply to both native and engineered antibodies, allowing efficient covalent linkage of cysteine residues and allowing conjugation with a DAR of one.

The present invention is based *inter alliae* on the provision of a versatile key rebridged protein conjugate having a degree of conjugation of 1, and a novel process for synthesizing the same. Said conjugate may conveniently lead to various drug conjugates

According to a first object, the present invention concerns a protein conjugate of formula (I):

Protein'-(L1)m-SH (I)

Wherein
Protein' is a re-bridged Protein or fragment thereof comprising interchain cysteine residues, whereas at least two of said interchain cysteines are bridged together through the sulfur (S) atoms of said cysteine residues by a group of formula (A-1) and optionally, the remaining interchain cysteine residues are bridged three by three by one or more groups of formula (A-2): where
the (A-1) group bridges together the -(L1)m-SH fragment and two sulfur atoms of two said cysteine residues;
the optional (A-2) group(s) bridge together three sulfur atoms of said remaining cysteine residues;
A represents a tri-valent group,
each * designates a covalent bond linking the A group of (A-1) and (A-2) with a sulfur atom of said cysteine residues;
** designates a covalent bond linking the A group of (A-1) with the (L1)m-SH fragment of conjugate (I);
SH is a thiol group;
L1 is an optional divalent group linking SH to the (A-1) group of Protein', said L1 preferably consisting of one or multiple successive units selected from the group consisting of : a linear or branched, saturated or unsaturated, C1-C60 alkylene group optionally interrupted and/or terminated on one or both sides by one or more chemical groups selected from -O-, -S-, - S(O)-, -SO₂-, -O-P(O)(OH)-O-, -C(O)-, -NH-, -NMe-, -C(O)NH-, -NHC(O)-, -NH-C(O)-NH, - O-C(O)-NH-, -O-C(O)-O-; C3-C8 cycloalkylene; C3-C6 heterocyclylene; 5 to 12 membered heteroarylene; C₆-C₁₂ arylene; amino acids; polypeptides; glycosylene; -(CH₂-CH₂-O-)ᵣ- groups; in which r is an integer ranging from 1 to 24 and/or optionally substituted by one or more of OH, CN, NH₂, CF₃, NHCONH₂, CONH₂, COOH, =O, =NH, glycosyl, C1-C12 alkyl, C6-C12 aryl, C5-C12 heteroaryl; -(CH₂-CH₂-O-)ᵣ-CH₃.
m is 0 or 1.

Said compound of formula (I) has a degree of conjugation of 1, meaning there is a single - (L1)m-SH fragment covalently bound to Protein'.

Prior art strategies are generally based on disulfide re-bridging agents performing the re-bridging of Cysteine thiols generated upon full or partial reduction of accessible disulfides.

By contrast, the present conjugates are based on a convenient site-selective tandem disulfide reduction-rebridging of all available cysteine residues of the hinge region and the provision of an additional reactive site for further functionalisation towards drug-antibody conjugates.

### Mixtures

According to the invention, the conjugates of formula (I) having a DoC=1 represent the majority (in number) compounds of the claimed mixtures.

According to the invention, the mixture comprises at least at least 50% (in number) of protein conjugates of formula (I), typically at least 60%, preferably at least 70%, more preferably at least 80%, most preferably at least 90% (in number).

One preferred feature of the mixture of the invention is that the mixture is essentially monodisperse with respect to the feature DoC=1 of said conjugate of formula (I).

Within the mixtures of said conjugates of formula (I) having a DoC=1, there may be minor side products, such as conjugates having a different degree of conjugation. Compounds having different DoC that are comprised in the mixture of the invention include partially conjugated compounds (ie) where less than one -(L1)m-SH fragment is covalently bound per Protein', that is DoC<1.

The term « mixture » thus encompasses the mixture comprising at least 50% (in number) of said conjugates of formula (I) having DoC=1 and other conjugates having different DoC.

Therefore, the term « mixture » as used herein refers to a mixture of at least 50% in number of conjugates of formula (I) having a DoC=1 and minority conjugates having DoC#1.

The nature of such mixtures may depend on the synthetic process, reactional conditions and/or reagents that may be used for synthesizing conjugates of formula (I).

### Protein'

Typically, Protein' may derive from Protein, where Protein is a naturally occurring protein or a synthetic protein, preferably a naturally occurring antibody or a synthetic antibody, such as an engineered antibody, herein referred to as Protein.

Said Protein typically comprises one or more polypeptide chains made of amino acids, it being provided that the cysteine residues may form covalent bonds through the formation of disulphide bridges, either within the same protein chain, or between different peptide chains within Protein. These are called herein « interchain cysteine residues ».

Typically, said naturally occurring or synthetic protein comprises two heavy chains and two light chains, where preferably each heavy chain comprising three cysteine residues and each light chain comprising one cystine residue in the hinge region. These chains are covalently bridged together by disulfide bonds created between the sulfur atom of the cysteine residues. Typically, the heavy chains are bridged together by the two interchain disulfide bridges created between two pairs of cysteine residues, and each heavy chain is further bridged to a light chain by a further disulfide bridge created between two cysteine residues.

According to an embodiment, said synthetic or naturally occurring protein comprises one or more aminoacid, such as peptide or a protein.

Typically, said Protein is a monoclonal antibody, such as human, humanized, mouse or chimeric antibodies. Typical antibodies include trastuzumab, bevacizumab, cetuximab, panitumumab, ipilimumab, rituximab, alemtuzumab, ofatumumab, gemtuzumab, brentuximab, ibritumomab, tositumomab, pertuzumab, adecatumumab, IGN101, INA01 labetuzumab, hua33, pemtumomab, oregovomab, minretumomab (CC49), cG250, J591, MOv-18, farletuzumab (MORAb-003), 3F8, ch14,18, KW-2871, hu3S193, IgN311, IM-2C6, CDP-791, etaracizumab, volociximab, nimotuzumab, MM-121, AMG 102, METMAB, SCH 900105, AVE1642, IMC-A12, MK-0646, R1507, CP 751871, KB004, III A4, mapatumumab, HGS-ETR2, CS-1008, denosumab, sibrotuzumab, F19, 81C6, pinatuzumab, lifastuzumab, glembatumumab, coltuximab, lorvotuzumab, indatuximab, anti-PSMA, MLN-0264, ABT-414, milatuzumab, ramucirumab, abagovomab, abituzumab, adecatumumab, afutuzumab, altumomab pentetate, amatuximab, anatumomab, anetumab, apolizumab, arcitumomab, ascrinvacumab, atezolizumab, bavituximab, bectumomab, belimumab, bivatuzumab, brontictuzumab, cantuzumab, capromab, catumaxomab, citatuzumab, cixutumumab, clivatuzumab, codrituzumab, conatumumab, dacetuzumab, dallotuzumab, daratumumab, demcizumab, denintuzumab, depatuxizumab, derlotuximab, detumomab, dinutuximab, drozitumab, duligotumab, durvalumab, dusigitumab, ecromeximab, edrecolomab, elgemtumab, emactuzumab, enavatuzumab emibetuzumab, enfortumab, enoblituzumab, ensituximab, epratuzumab, ertumaxomab, etaracizumab, farletuzumab, ficlatuzumab, figitumumab, flanvotumab, futuximab, galiximab, ganitumab, icrucumab, igovomab, imalumab, imgatuzumab, indusatumab, inebilizumab, intetumumab, iratumumab, isatuximab, lexatuzumab, lilotomab, lintuzumab, lirilumab, lucatumumab, lumretuzumab, margetuximab, matuzumab, mirvetuximab, mitumomab, mogamulizumab, moxetumomab, nacolomab, naptumomab, narnatumab, necitumumab, nesvacumab, nimotuzumab, nivolumab, nofetumomab, obinutuzumab, ocaratuzumab, ofatumumab, olaratumab, onartuzumab, ontuxizumab, oportuzumab, oregovomab, otlertuzumab, pankomab, parsatuzumab, pasotuxizumab, patritumab, pembrolizumab, pemtumomab, pidilizumab, pintumomab, polatuzumab, pritumumab, quilizumab, racotumomab, ramucirumab, rilotumumab, robatumumab, sacituzumab, samalizumab, satumomab, seribantumab, siltuximab, sofituzumab, tacatuzumab, taplitumomab, tarextumab, telisotuzumab, tenatumomab, teprotumumab, tetulomab, ticilimumab, tigatuzumab, tisotumab, tositumomab, tovetumab, tremelimumab, tucotuzumab, ublituximab, ulocuplumab, urelumab, utomilumab, vadastuximab, vandortuzumab, vantictumab, vanucizumab, varlilumab, veltuzumab, vesencumab, volociximab, vorsetuzumab votumumab, zalutumumab, zatuximab, zilovertamab, combination and derivatives thereof., as well as other monoclonal antibodies targeting HER2, Nectin-4, tissue factor, Trop-2, FRα, ROR1, EGFR, cMET, B7-H3, CAI 25, CAI 5-3, CAI 9-9, L6, Lewis Y, Lewis X, alpha fetoprotein, CA 242, placental alkaline phosphatase, prostate specific antigen, prostate specific membrane antigen, prostatic acid phosphatase, epidermal growth factor, MAGE-I, MAGE-2, MAGE-3, MAGE-4, transferrin receptor, p97, MUCI, CEA, gplOO, MARTI, IL-2 receptor, CD20, CD52, CD33, CD22, human chorionic gonadotropin, CD38, CD40, mucin, P21, MPG, and Neu oncogene product.

According to an embodiment, Protein' may derive from a naturally occurring antibody comprising two heavy chains and two light chains in which the two heavy chains are bridged together by two disulfide bridges, and each heavy chain is bridged to a light chain by a disulfide bridge, and Protein' differs from said naturally occurring antibody in that in Protein' the heavy and light chains are re-bridged through one (A-1) and two (A-2) groups, in particular the two heavy chains are bridged together through (A-1) each is further bridged with a light chain through a (A-2) group.

According to an alternative embodiment, Protein' may derive from an engineered antibody (or so called "mutant" antibody).

A particular illustrative embodiment is an antibody comprising two heavy chains and two light chains wherein 6 out of 8 interchain cysteines, which typically form the interchain disulfide bonds, were mutated into any other amino acid, either the same or different, such as lysine, arginine, tyrosine, phenylalanine, tryptophan, histidine, serine, threonine. Typically, the resulting engineered antibody comprised a single interchain disulfide bond. Illustrative examples of such engineered antibody include trastuzumab (HC-C229S, HC-C223S, LC-C214S) mutant and J591 (HC-C220S, HC-C229S, LC-C214S) mutant. Typically in this embodiment, Protein' differs from said engineered antibody in that in Protein' the two heavy chains are re-bridged together through one (A-1) group, or two cysteines on the same heavy chain are rebridged.

Another illustrative embodiment is an engineered antibody wherein two additional cysteine groups have been introduced into the protein sequence.

Illustrative examples of such engineered antibody include Thiomab^{®} described in US7,521,541, as well as antibodies with cysteines introduced through following point mutations: LC-V110C, HC-A114C, HC-D265C, HC-S239C, HC-S442C, HC-N325C, HC-L328C.

Typically in this embodiment, Protein' differs from said engineered antibody in that the two introduced cysteine groups are bridged through one (A-1) group.

### The conjugates

According to the invention, the conjugates of formula (I) or (II) as defined herein may be present in the form of different regioisomers.

This is because Protein generally comprises a number of cysteine residues that can be available for the rebridging through (A-1) and optionally (A-2).

The group (A-1) (and optionally (A-2)) may rebridge different cysteine groups within Protein' (ie) cysteine groups that may be located at various sites within Protein'.

In other words, various combinations of rebridged cysteine groups are possible and may lead to different conjugates present in the conjugates. As an illustrative example, (A-1) may bridge together two sulfur atoms that are each located on a different heavy chain, or it may bridge together two sulfur atoms wherein one is located on a light chain and the other is located on a heavy chain.

Similarly, any (A-2) group, when present may involve any three of the available cysteine groups.

The combinations result in different conjugates that all have a DoC=1, but that differ by the sites of attachment of the (A-1) (and optionally any (A-2)) group within Protein'. Each combination of covalently bound cysteine residues thus leads to a regioisomer having a DoC =1.

Thus, the term "conjugates" thus encompasses and also refers to all regioisomers thereof, and the mixtures consisting of such regioisomeric conjugates having a Doc=1.

It is also to be understood that said conjugates may be prepared alongside byproducts. These byproducts may add up to less than 50% (number) of the mixture, preferably less than 40%, more preferably less than 30%, still more preferably less than 20%, in particular less than 10% (number) of the mixture.

For example, byproducts may comprise conjugates with different DoC (e.g. DoC=0, or DoC>1). These byproducts are not regioisomers of the DoC=1 species, since they have a different number of A1 and A2 groups, as well as other groups, formed as a result of side reactions.

Therefore, according to the invention, the term "mixture of conjugates" particularly refers to a mixture comprising at least 50% (number) of said conjugates having a DoC=1, preferably at least 60%, more preferably at least 70%, still more preferably at least 80%, in particular at least 90% (number) of the mixture, where said DoC=1 conjugates are regioisomers.

In the sense of the invention, the term "regioisomer" thus refers to isomeric conjugates, where the site of attachment of (A-1) (and optionally any (A-2)) with cysteine residues may vary on the backbone of Protein'.

As discussed above, the conjugates of the invention may thus be "heterogeneous" in that the fragment that is covalently bound to said Protein' may be attached to different attachment sites of Protein'.

Without being bound by any theory, it is expected that the heterogeneity in the sense of the invention may be an important feature to overcome some of the drug resistance mechanisms (as in the organism becomes resistant to only one of the many structural/regional variants). Moreover, heterogeneity in the sense of the invention prevents the formation of hydrophobic clusters on the surface of the protein and therefore has a positive effect on the pharmacokinetics of the conjugates (Nature biotechnology, vol.33, 7, 694-696, 2015).

Representative conjugates of formula (I) include those described in the examples below, preferably :

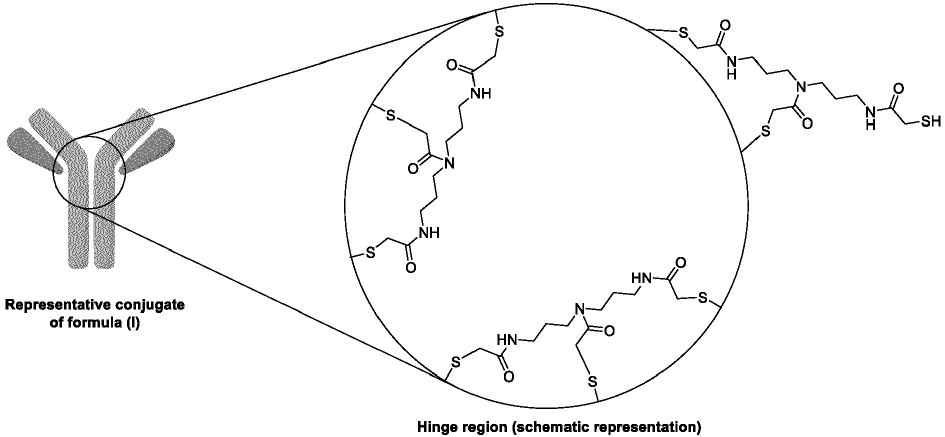

### The A groups

The trivalent A groups may be typically chosen from linear, branched or cyclic trivalent moieties, comprising 1 to 9 nitrogen atoms and from 0 to 30 carbon atoms, said moiety comprising three terminal functions of formula:

-NH-(C=O)-CH₂-

Where the terminal -CH₂- group is attached to the * or ** bond

Where * and ** are defined as in formula (A-1) and (A-2).

According to a particular embodiment, A may include the following groups: 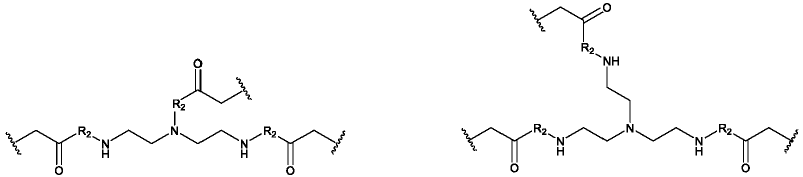 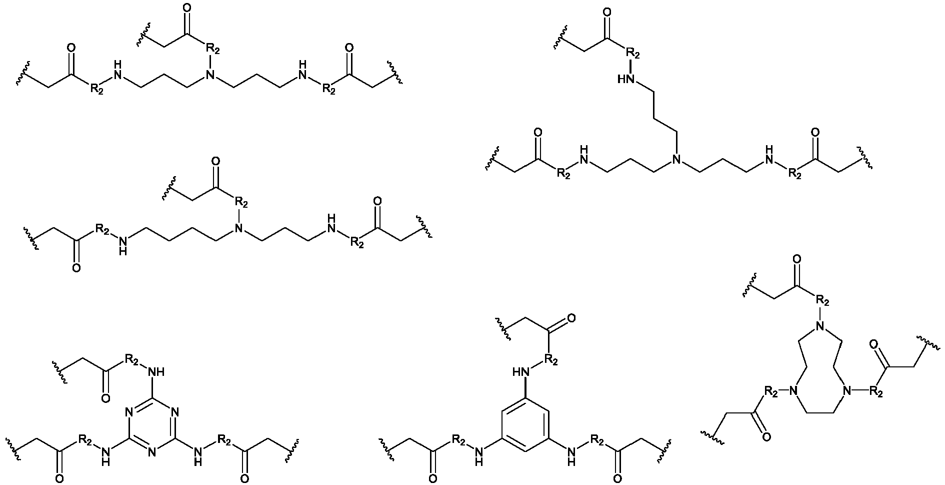 where R₂ is an optional spacer unit selected from the group consisting of a C6-C12 arylene; a linear or branched, saturated or unsaturated C1-C60 alkylene group optionally interrupted by one or more chemical groups selected from -O-, -S-, -S(O)-, -SO₂-, -O-P(O)(OH)-O-, - C(O)-, -NH-, -C(O)NH-, -NHC(O)-, -C(O)NR-, -NRC(O)-, heteroarylene, arylene, glycosylene, an -O-(CH2-CH2-O-)ᵣ -, -NH-(CH₂-CH₂-O-)ᵣ- , -(-O-CH₂-CH₂)ᵣ , -(CH₂-CH₂-O-)ᵣ- groups, amino acids and peptide residues, where R is H or a C1-C6 alkyl and r is an integer ranging from 1 to 24.

Typically, illustrative A groups include:

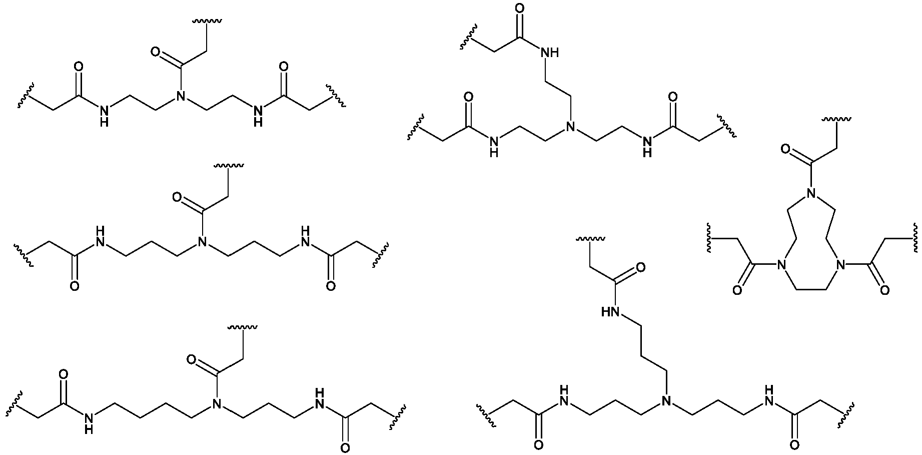

According to a further object, the present invention concerns the process of preparation of the protein conjugate of formula (I), said process comprising the step of reacting a conjugate of formula (IV) :

Protein'-Hal (IV)

With a dithiol compound, a hydrosulfide salt or sulfide salt, where Protein' is defined as above and Hal is a halogen atom chosen from F, Cl, I and Br.

Suitable dithiol compounds include compounds of formula HS-(L1)ₘ-SH where L1 and m are defined as in formula (I).

Representative dithiol compounds include :

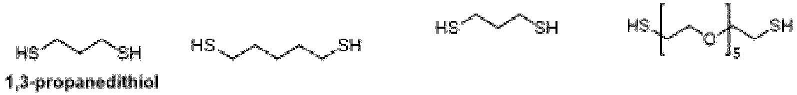

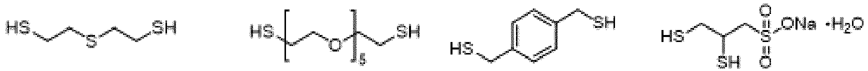

Hydrosulfide and sulfide salts include sodium hydrosulfide (NaHS), sodium sulfide (Na₂S), potassium hydrosulfide (KHS), potassium sulfide (K₂S), ammonium hydrosulfide (NH₄HS), ammonium sulfide ((NH₄)₂S).

This reaction is typically conducted as a nucleophilic substitution (SN2), in the presence of large equivalents of the dithiol compound (such as more than 100 equivalents), with respect to the compound (IV). Generally, it is conducted in conditions compatible with Protein', such as pH comprised between 7 and 9, preferably between 8 and 8.5, at a temperature comprised between 20 and 40°C, preferably at room temperature.

Typically, the reaction may be conducted in a buffered medium such as TBS, PPB, PBS, HEPES medium.

According to an embodiment, the process of preparing the protein conjugate (I) further comprises preparing the conjugate of formula (IV) by reducing disulfide bridges that are present in Protein, and re-bridging the reduced sulfur atoms of Protein, said reducing and re-bridging comprising :
Reacting Protein with a reducing agent, and with a re-bridging agent of formula (B): 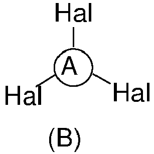
where Protein is a natural or synthetic protein or fragment thereof;
A is defined as in anyone of claims 1 to 7; and
Hal is a halogen atom chosen from F, Cl, I and Br.

The reducing and rebridging steps may be conducted separately in sequence or as a one pot reaction. Preferably, they may be performed as a tandem one-pot reaction (denoted as "rr").

Generally, these steps may be conducted in conditions compatible with Protein', such as pH comprised between 7 and 9, preferably between 8 and 8.5, at a temperature comprised between 20 and 40°C, preferably at room temperature.

Typically, the reaction may be conducted in a buffered medium such as TBS, PPB, PBS, HEPES medium.

Reaction time may be comprised between 1 and 10 hours, preferably between 1 and 5 hours.

The concentration of Protein in the reactional mixture may be adjusted; advantageously it may be comprised between 6.0 and 8.0 g/L.

The reducing and rebridging reagents may be preferably used in excess with espect to Protein, typically between 5 and 10 equivalents.

Said reducing agent may be for example Tris(2-carboxyéthyl)phosphine hydrochloride (TCEP), dithiothreitol (DTT) or 2-mercaptoethanol (BME).

For the reducing step, preferred conditions include 37 °C, 1.5 h, 5-10 equiv. TCEP.

Suitable rebridging reagents may be chosen from halogen acetamides, such as :

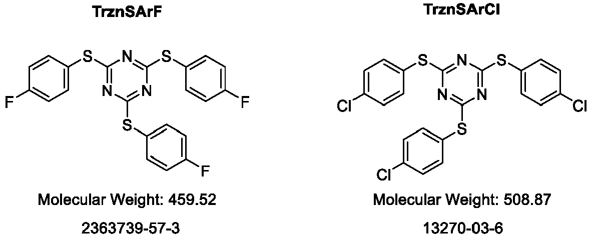

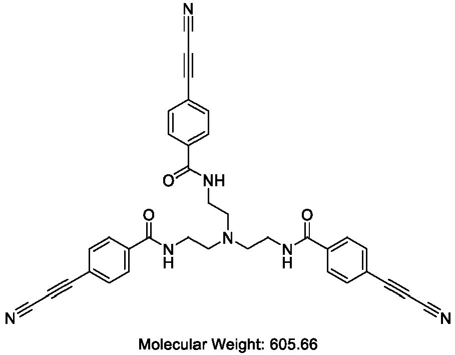

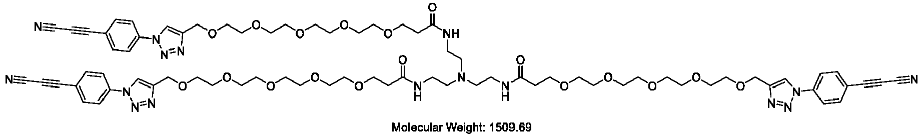

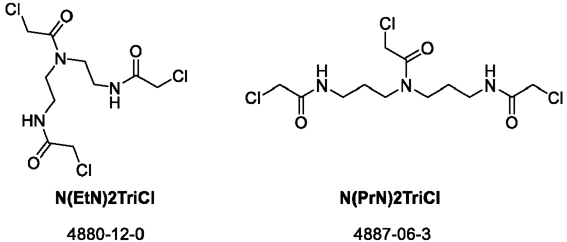

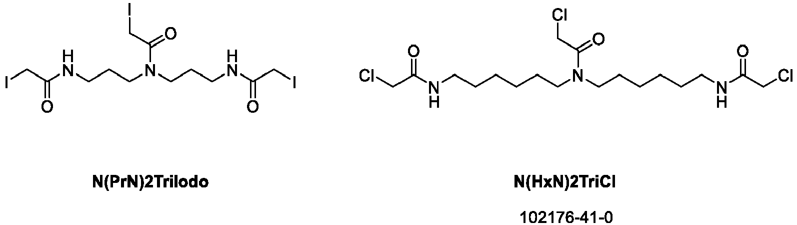

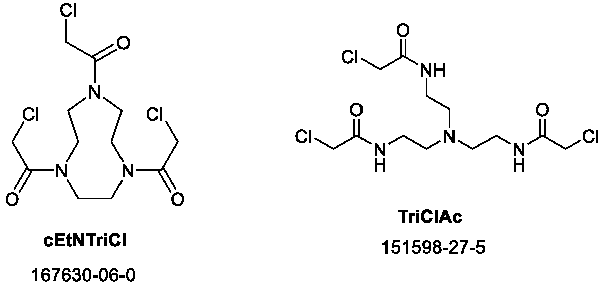

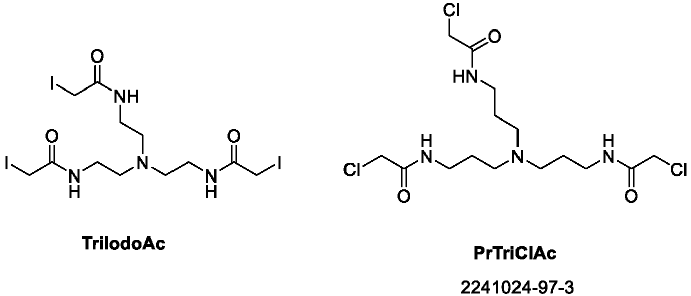

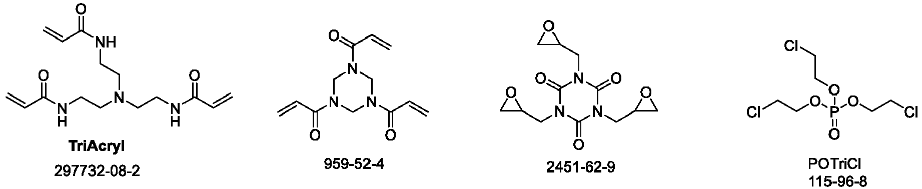

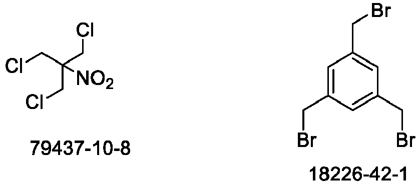

Chloroacetamides are preferred, and more particularly:

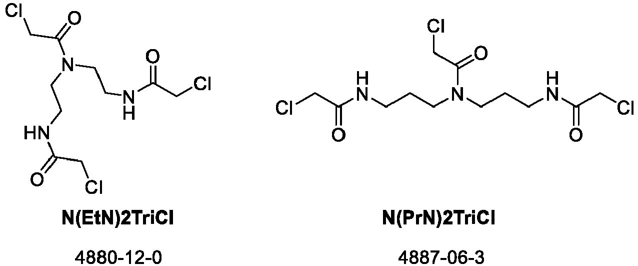

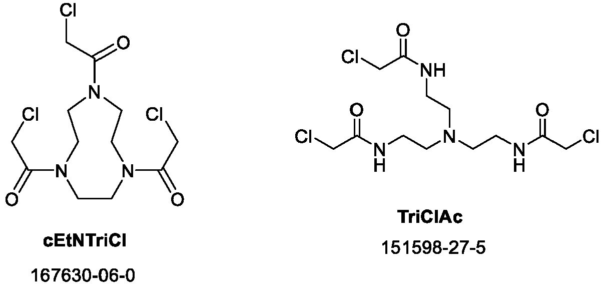

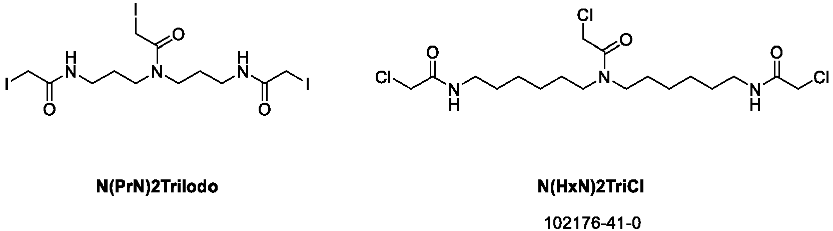

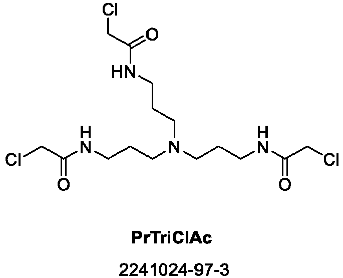

Some compounds of formula (B) are novel and are part of the present invention. In particular, according to a further object, the present invention concerns the compound of formula (B-1):

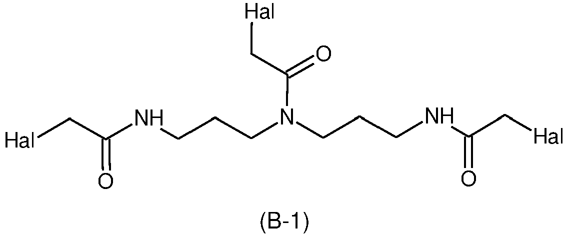

Where Hal is a halogen atom.

The process thus involves a convenient tandem/one-step reduction and rebridging.

Advantageously, it does not require the use of organic co-solvent. Previously reported re-bridging reagents commonly showed limited solubility in aqueous media and typically require organic co-solvents to facilitate the reaction. This ultimately raises the risk of causing denaturation to the antibody and impacts its therapeutic properties.

Further, the process of the invention may involve a low number of purification steps (Biospin). In particular, chromatographic purification of rebridging agent may not be required.

It provides inexpensive and scalable one-step synthesis.

The key conjugates of formula (I) and the mixtures thereof provide an additional functionality for payload-linker conjugation.

The modularity that is introduced potentially allows the attachment of a variety of payloads. This broadens the diversity of payloads that can be attached to the antibody through different types of conjugation chemistries.

They thus represent versatile key intermediate conjugates towards a diversity of antibody-drug conjugates (ADCs).

According to a further object, the present invention also concerns a protein conjugate of formula (II):

Protein'-(L1)ₘ-S-R'-L2-Payload (II)

Wherein
Protein', L1 and m are defined as in formula (I),
R' is a group formed as a result of a reaction between a thiol and a thiol-reactive group, such as maleimide, 3-arylpropiolonitrile, bromo-maleimide, chloroacetamide, bromoacetamide, iodoacetamide, acrylamide, vinyl sulfone, pyridyl disulfide, alpha-halo carbonyl, vinyl pyridine;
L2 is a divalent linker connecting together the R' and Payload moieties;
Payload is a molecule which exerts biological activity.

Compounds of formula (II) may be referred to as « antibody-drug conjugates » (ADC).

### Payload

Payload may be chosen from drug or imaging agents, oligonucleotides, chelating ligands capable of complexing with radionuclides, cytotoxic drugs and antineoplastic agents. According to an embodiment, Payload may be chosen from drugs, such as cytotoxic drugs and antineoplasic agents.

Suitable drugs include in particular:
- dolastatins such as MMAE, MMAF, MMAD;
- maytansins such as DM1 and DM4;
- antracyclins such as doxorubicin, nemorubicin and PNU-159682;
- calicheamicins;
- duocarymycins such as CC-1065 and duocarmycin A;
- pyrrolobenzodiazepines;
- pyrrolobenzodiazepine dimers;
- indolino-benzodiazepines;
- indolino-benzodiazepine dimers;
- amanitins such as α-amanitin, β-amanitin, γ-amanitin, ε-amanitin;
- irinotecan derivatives;
- exatecan derivatives;
- exotoxins such as diphtheria toxin, shiga toxin, or subunits thereof;
in particular:
- dolastatins such as MMAE, MMAF, MMAD;
- maytansins such as DM1 and DM4;
- antracyclins such as doxorubicin, nemorubicin and PNU-159682;
- calicheamicins;
- duocarymycins such as CC-1065 and duocarmycin A;
- pyrrolobenzodiazepines;
- pyrrolobenzodiazepine dimers;
- indolino-benzodiazepines;
- indolino-benzodiazepine dimers;
- amanitins such as α-amanitin, β-amanitin, γ-amanitin, ε-amanitin; and
- irinotecan derivatives;
- exatecan derivatives.
- vincristine derivatives.

Suitable oligonucleotides include silencing RNA, microRNA, antisense oligonucleotides, DNA, or RNA oligomers. Typically they are between 10 and 5000 nucleotides in length, more particularly, between 20 and 200.

Imaging agents include detection agents, biological markers, tracing agents, such as fluorophores, dyes, radioactive tracers.

### R' spacers, L2 linkers

R' may include the following spacers: 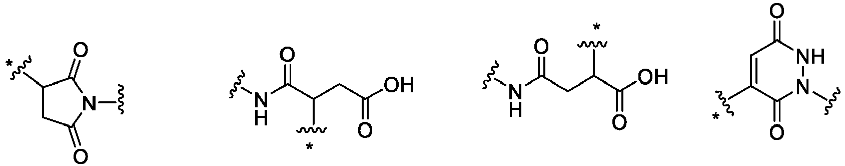 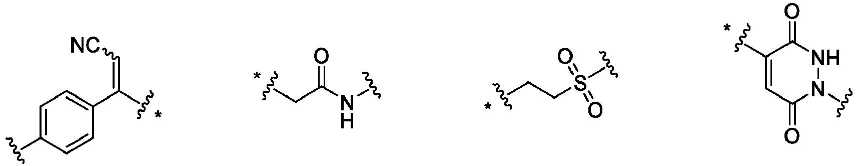
* designates a bond linking the R' group with a sulfur atom;
L2 and L1 may be the same or different, typically different.

According to an embodiment, L2 may consist of one or multiple successive units selected from the group consisting of a linear or branched, saturated or unsaturated, C1-C60 alkylene group optionally interrupted and/or terminated on one or both sides by one or more chemical groups selected from -O-, -S-, -S(O)-, -SO₂-, -O-P(O)(OH)-O-, -C(O)-, -NH-, -NMe- , -C(O)NH-, -NHC(O)-, -NH-C(O)-NH, -O-C(O)-NH-, -O-C(O)-O-; C3-C8 cycloalkylene; C3-C8 heterocyclylene; 5 to 12 membered heteroarylene; C6-C12 arylene; amino acids; polypeptides; glycosylene; -(CH₂-CH₂-O-)ᵣ- groups; in which r is an integer ranging from 1 to 24 and/or optionally substituted by one or more of OH, CN, NH₂, CF₃, NHCONH₂, CONH₂, COOH, =O, =NH, glycosyl, C1-C12 alkyl, C6-C12 aryl, C5-C12 heteroaryl; - (CH₂-CH₂-O-)ᵣ-CH₃.

In certain cases, L2 may also include units for the traceless release of the Payload, such as para-amino-benzyl or enzyme-cleavable fragments like peptides and glucosides

The -S-R'-L2-Payload fragments may be diversely chosen and are not limited according to the invention. Many such fragments have been described and developed in existing conjugates. Representative known fragments include:

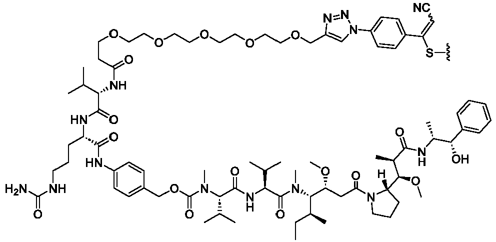

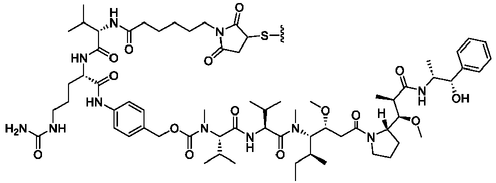

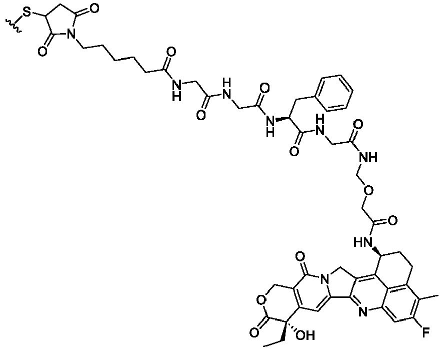

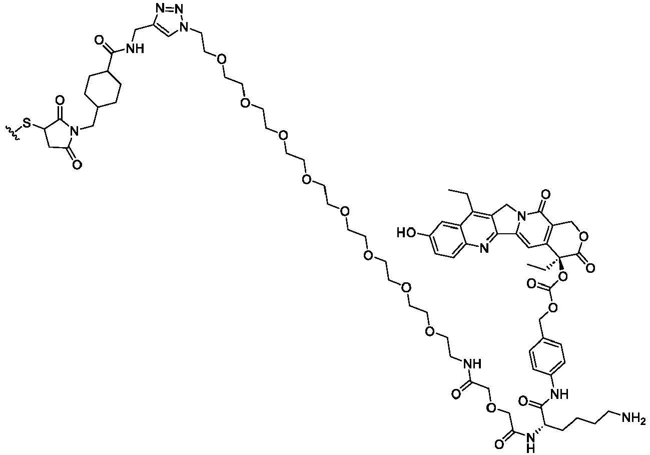

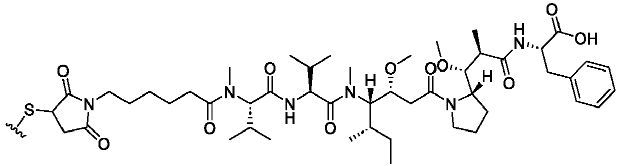

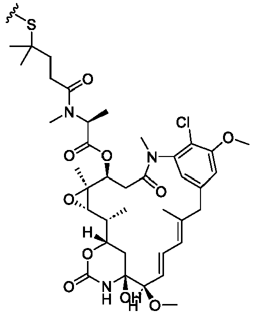

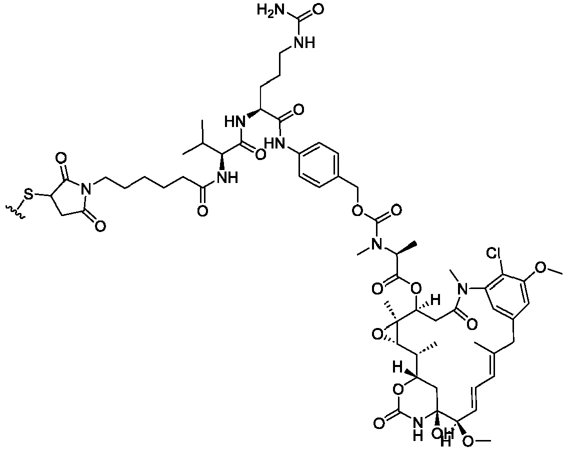

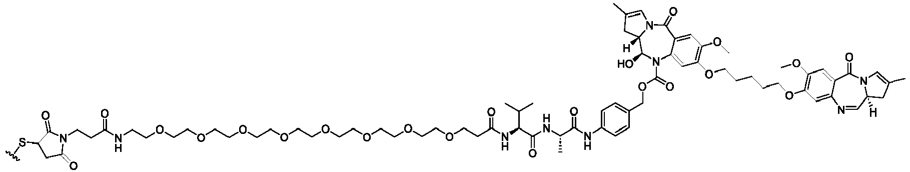

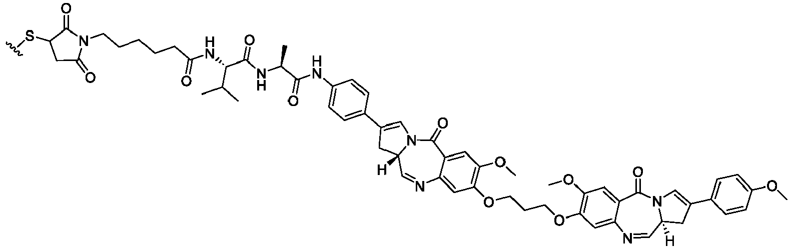

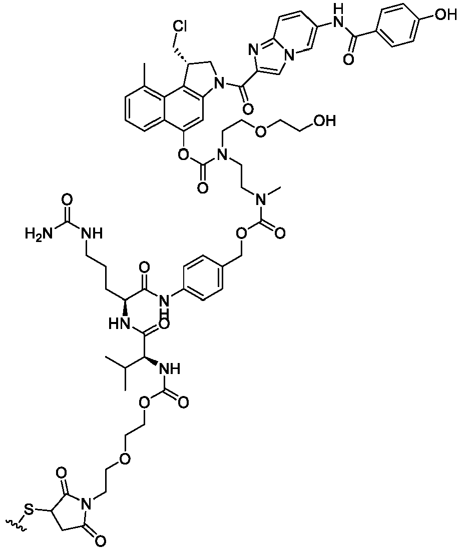

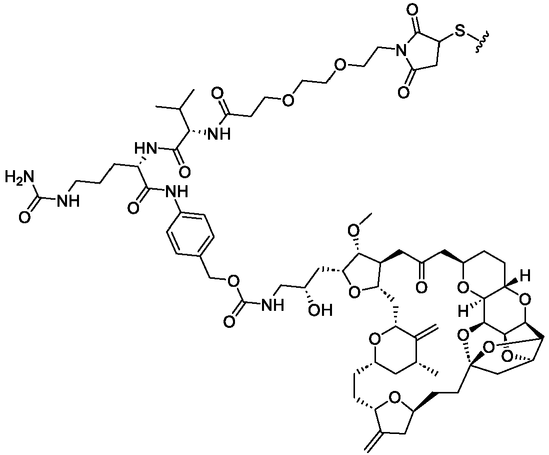

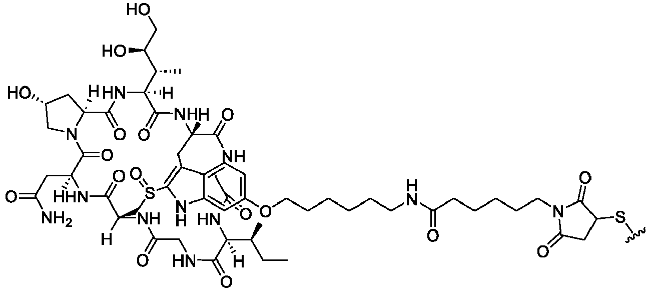

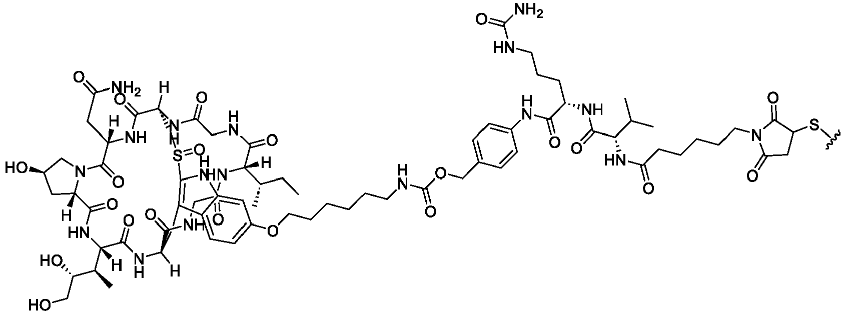

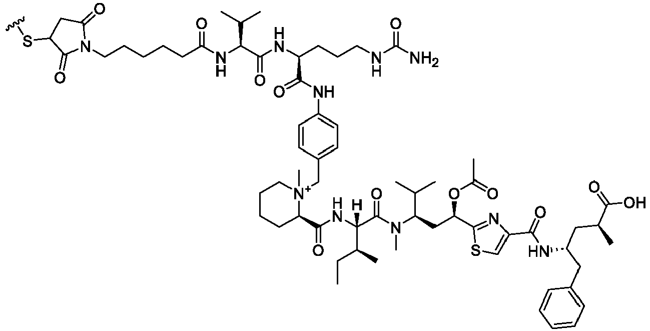

According to a further object, the present invention also concerns a pharmaceutical composition comprising a conjugate of formula (II) or mixture thereof as defined above and one or more pharmaceutically excipient.

According to a still further object, the present invention also concerns a conjugate of formula (II) or the mixture thereof as defined above, where Payload is a cytotoxic drug or antineoplastic agent, or an imaging agent for use for treating, preventing and/or diagnosing cancer.

According to a further object, the present invention also concerns a method of treating, preventing and/or diagnosing a condition or disorder chosen from the group consisting in tumor, cancer, autoimmune disease, or infectious disease in a patient in the need thereof, comprising administering a conjugate of formula (II) as defined above to said patient.

The identification of those subjects who are in need of treatment of herein-described diseases and conditions is well within the ability and knowledge of one skilled in the art. A clinician skilled in the art can readily identify, by the use of clinical tests, physical examination, genetic tests and medical/family history, those subjects who are in need of such treatment.

The therapeutically effective amount of conjugates of formula (II) can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances. The amount of conjugates of formula (II) which is required to achieve the desired biological effect, will vary depending upon a number of factors, including the dosage of the conjugates to be administered, the chemical characteristics (e.g. hydrophobicity) of the compounds employed, the potency of the compounds, the type of disease, the diseased state of the patient and the route of administration.

"Pharmaceutically" or "pharmaceutically acceptable" refer to molecular entities and compositions that do not produce an adverse, allergic or other untoward reaction when administered to an animal, or a human, as appropriate.

As used herein, "pharmaceutically acceptable excipient" includes any diluents, adjuvants, excipients, or vehicles, such as preserving agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

In the context of the invention, the term "treating" or "treatment", as used herein, means reversing, alleviating, inhibiting the progress of, or preventing the disorder or condition to which such term applies, or one or more symptoms of such disorder or condition.

"Therapeutically effective amount" means an amount of a compound/medicament according to the present invention effective in producing the desired therapeutic effect.

According to the invention, the term "patient", or "patient in need thereof", is intended for a human or non-human mammal affected or likely to be affected with cancer. Preferably, the patient is a human.

The compositions may conveniently be administered in unit dosage form and may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier.

### FIGURES

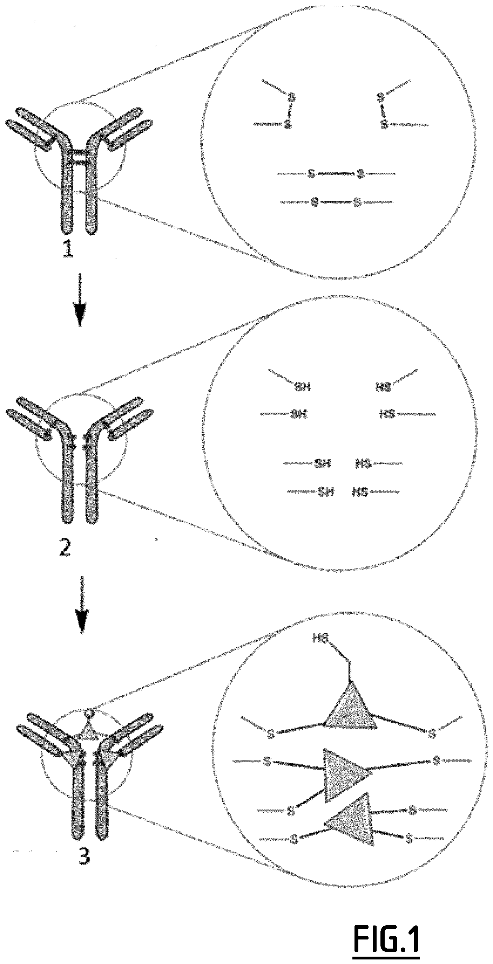

Figure 1 schematically represents the synthesis towards an illustrative rebridged protein according to the invention (MonoThiomab ^{®}), wherein (1) represents a native IgG1 or IgG4 comprising 8 cysteine groups linked together through their sulfide atoms, thus comprising 4 disulfide bonds.

Following reduction, the sulfide atoms are reduced leading the fully reduced (2) IgG1 or IgG4 comprising 8 thiol (-S) groups. Said reduced protein (2) is then reacted with a suitable re-bridging reagent (such as Tri-Acc), followed by a suitable dithiol (such as 1,3-propanedithiol) leading to the rebridged protein (3), wherein only one thiol group is free, and all remaining thiol groups are rebridged together.

In Figure 1, the rebridged (3) as represented is only one of the possible isomers that can be obtained as the rebridging may involve different thiol groups at any of their position and the free reactive thiol group can be issued from any of the cysteine groups.

Figure 2 illustrates a representative synthesis scheme according to the invention, starting from a native antibody, leading to the re-bridged antibody following reduction-rebridging and dithiol substitution reactions (1 and 2), said re-bridged antibody having only one reactive thiol group (-SH), which then can be conjugated with a payload such as MC-VC-PAB-MMAE to lead to the conjugate comprising said re-bridged antibody with one payload having a DAR of one.

Figure 3 is an illustration of the synthesis scheme with an expanded view of the modifications of the hinge region involving interchain cysteine residues. Again, only one regioisomer is represented and alternative regioisomers can be obtained depending on the sulfur atoms that are involved in the rebridging reaction.

Figure 4 is a chromatogram typical of the mixture of protein conjugates (compound 22) comprising at least 50% (in number) of protein conjugates of formula (II) of the invention as obtained by HIC.

### EXAMPLES

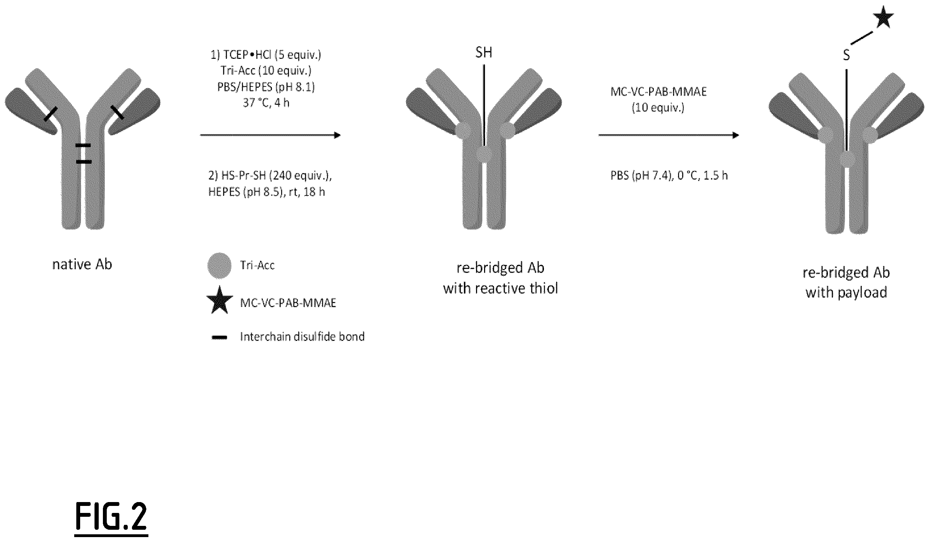

An illustrative reaction sequence is shown in Figure 2 was conducted.

Synthesis was conducted with trastuzumab as Protein.

Upon complete reduction of all four disulfide bonds in the IgG hinge region, the complete re-bridging was envisaged via the incorporation of three molecules of trichloro acetamides (Tra-CI). The remaining chloro acetamide in Tra-CI was engaged in an SN2 reaction with a dithiol affording the corresponding Tra-SH species.

The latter was conjugated with MC- or APN-vc-PAB-MMAE providing the final ADCs.

Suitable reagents and conditions for the reduction and rebridging steps include : (1) TCEP and hydrochloride acid (5 equivalents), Tri-Acc (10 equivalents) in PBS/HEPES (pH 8.1) at 37°C, during 4 hours.

All re-bridging reactions were carried out with an initial [Trastuzumab] = 7.6 - 8.1 g/L, using 10 equiv. of TCEP for disulfide reduction, which were not discarded before adding a re-bridging reagent, unless otherwise stated. After re-bridging reactions, the mixtures were purified over Biospin to remove excess reagents prior to adding 1,3-propanedithiol.

| Entry | Rebr agent | equiv | Rebr buffer, pH | Rebr T (°C) | Rebr time (h) | A1% |
|---|---|---|---|---|---|---|
| 1 | TriClAc | 30 | H, 8.5 | 25 | 25 | 52.4 |
| 2 | TriClAc | 30 | P+H, 8.3 | 25 | 25 | 55.4 |
| 3 | TriClAc | 20 | P+H, 8.3 | 25 | 25 | 53.0 |
| 4 | TriClAc | 120 | P+H, 8.3 | 25 | 25 | 53.9 |
| 5 | TriClAc | 30 | P+H, 8.3 | 25 | 25 | 50.3 |
| 6 | TriClAc | 30 | P+H, 8.3 | 37 | 3.5 | 52.6 |
| 7 | TriClAc | 30 | H, 8.5 | 37 | 3.5 | 44.6 |
| 8 | TriClAc | 30 | P+H, 7.9 | 37 | 3.5 | 34.8 |
| 9 | TriClAc | 30 | P+H, 8.3 | 37 | 4 | 52.7 |
| 10 | TriClAc | 30 | P+H, 8.3 | 37 | 4 | 50.5 |
| 11 | TriClAc | 30 | P+H, 8.3 | 37 | 4 | 46.6 |
| 12 | TriClAc | 30 | P+H, 8.3 | 37 | 3.5 | 44.9 |
| 13 | PrTriClAc | 30 | P+H, 8.3 | 37 | 3.5 | 54.6 |
| 14 | N(EtN)2TriCl | 30 | P+H, 8.3 | 37 | 4 | 52.4 |
| 15 | N(PrN)2TriCl | 30 | P+H, 8.3 | 37 | 4 | 62.0 |
| 16 | N(PrN)2TriCl | 10 | P+H, 8.1 | 37 | 4 | 65.4 |
| 17 | cNEtTriCl | 10 | P+H, 8.1 | 37 | 4 | 62.2 |
| 18 | N(PrN)2TriCl | 10 | P+H, 8.1 | 37 | 4 | 59.6 |
| 19 | N(PrN)2TriCl | 10 | P+H, 8.1 | 37 | 4 | 68.01 |
| 20 | N(HxN)2TriCl | 10 | P+H, 8.1 | 37 | 4 | 51.7 |

| | | | | | | |
|---|---|---|---|---|---|---|
| A1%: area percentage of DAR1. P: PBS. H: HEPES. | | | | | | |

### Experimental Part

### General

The following chemicals investigated as re-bridging reagents, namely tris(2-chloroethyl) phosphate (CAS = 115-96-8), 1,1',1"-(1,3,5-triazinane-1,3,5-triyl)tris(prop-2-en-1-one) (CAS = 959-52-4), 1,3,5-tris(oxiran-2-ylmethyl)-1,3,5-triazinane-2,4,6-trione (CAS = 2451-62-9) were purchased from TCI Chemicals and used as such. Likewise, 1,3-dichloro-2-(chloromethyl)-2-nitropropane (CAS = 79437-10-8) and 1,3,5-tris(bromomethyl)benzene (CAS = 18226-42-1) were purchased from Sigma-Aldrich and used as such.

### General Procedure A for the synthesis of trichloro acetamides

To a solution of triamine (1.0 equiv.) in DCM (1 mL/mmol) was added 6 M K₂CO₃ (4.94 equiv., 2.75 mL, 16.5 mmol) in H₂O and the mixture was cooled to 0 °C. Upon vigorous stirring, chloroacetyl chloride (3.3 equiv.) was added dropwise, and the mixture was allowed to stir at room temperature until full conversion of the triamine as indicated by TLC. The mixture was diluted with water (5 mL/mmol of amine) and extracted with DCM (3 × 5 mL/mmol of amine). Subsequently, the combined organic layer was washed with brine (2 × 5 mL/mmol of amine), dried over Na₂SO₄, and concentrated under reduced pressure. The concentrate was filtered over a pad of silica and washed with DCM/MeOH 9:1. Where necessary, the crude products were purified via flash chromatography with DCM/MeOH 10:0 to 8:2.

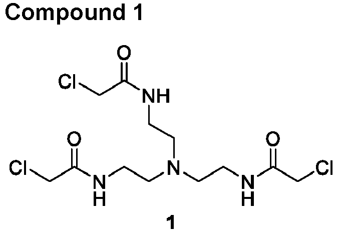

Compound 1 (*N*,*N*',*N*"-(nitrilotris(ethane-2,1-diyl))tris(2-chloroacetamide)) was synthesized following General procedure A using *N*¹,*N*¹-bis(2-aminoethyl)ethane-1,2-diamine as the triamine component.

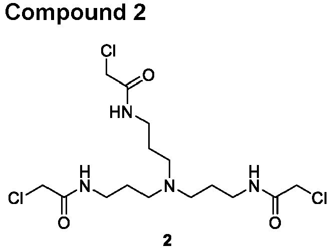

Compound **2** (*N*,*N*',*N*"-(Nitrilotris(propane-3,1-diyl))tris(2-chloroacetamide)) was synthesized following General procedure A using *N*¹,*N*¹-bis(3-aminopropyl)propane-1,3-diamine as the triamine component.

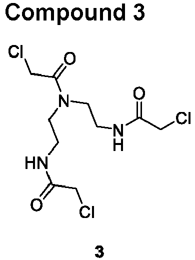

Compound **3** (2-Chloro-*N*,*N*-bis(2-(2-chloroacetamido)ethyl)acetamide) was synthesized following General procedure A using *N¹*-(2-aminoethyl)ethane-1,2-diamine as the triamine component.

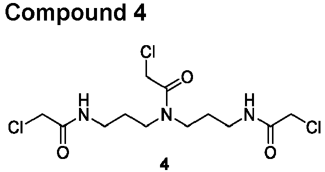

Compound **4** (2-Chloro-*N*,*N*-bis(3-(2-chloroacetamido)propyl)acetamide) was synthesized following General procedure A using *N¹*-(3-aminopropyl)propane-1,3-diamine as the triamine component.

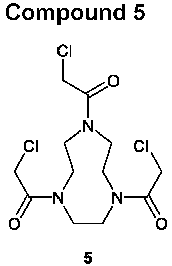

Compound 5 (1,1',1"-(1,4,7-Triazonane-1,4,7-triyl)tris(2-chloroethan-1-one)) was synthesized following General procedure A using 1,4,7-triazonane as the triamine component.

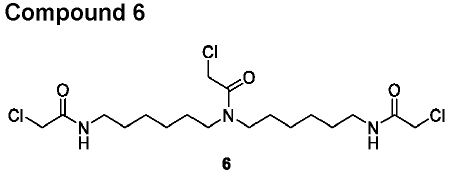

Compound **6** (2-Chloro-*N*,*N*-bis(6-(2-chloroacetamido)hexyl)acetamide) was synthesized following General procedure A using *N¹*-(6-aminohexyl)hexane-1,6-diamine as the triamine component.

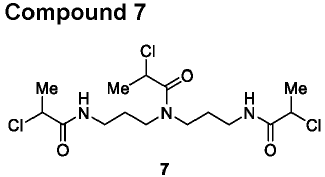

Compound **7** (2-Chloro-*N*,*N*-bis(6-(2-chloroacetamido)hexyl)acetamide) was synthesized following General procedure A using *N¹*-(3-aminopropyl)propane-1,3-diamine as the triamine component.

### Synthesis of compound 8 (2-((2-(2-chloroacetamido)ethyl)thio)-N,N-bis(3-(2-chloroacetamido)propyl)acetamide)

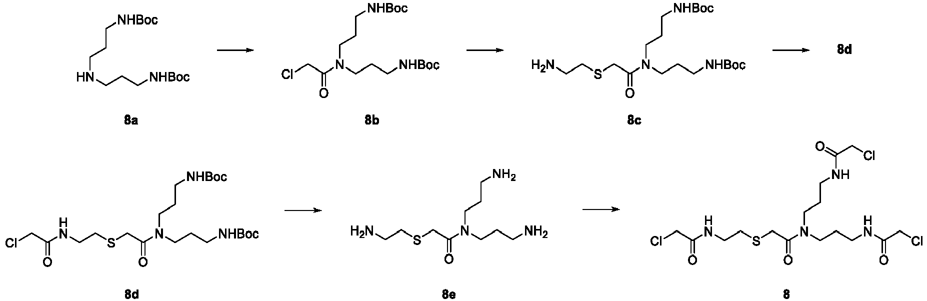

Compound **8b** was synthesized following General procedure A using compound **8a** (di-tert-butyl (azanediylbis(propane-3,1-diyl))dicarbamate) as the triamine component. To a suspension of compound **8b** (1.0 equiv.) in PBS was added K₂CO₃ (1.1 equiv.) and next 2-aminoethane-1-thiol (1.2 equiv.) at rt. The mixture was set to 40 °C for 1 h to give **8c** and the mixture was directly engaged into the synthesis of compound **8d** following General procedure A. To a solution of compound **8d** in CDCl₃ was added 10% TFA and the mixture was set to 37 °C for 30 min to give **8e.** After evaporation of the volatiles under reduced pressure, compound **8** was synthesized following General procedure A.

### Synthesis of compound 9 (2-((2-(2-chloroacetamido)ethyl)thio)-N,N-bis(3-(2-((2-(2 chloroacetamido)ethyl)thio)acetamido)propyl)acetamide)

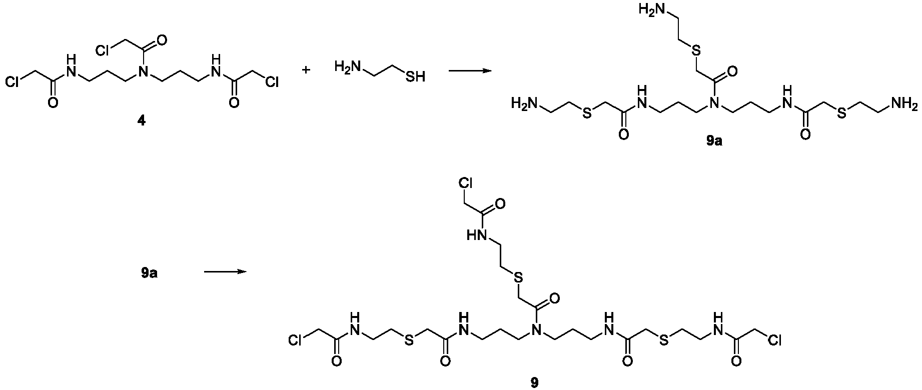

To a solution of compound **4** (1.0 equiv., 10 mM) in PBS was added 2-aminoethane-1-thiol (10.6 equiv., 0.2 M) as a solution in PBS at rt under vigorous stirring. After 5 minutes the mixture was purified via HPLC to give compound **9a** as a yellow oil. Compound **9** was synthesized following General procedure A from compound **9a.**

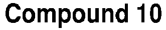

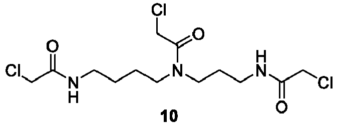

Compound **10** (2-chloro-N-(4-(2-chloroacetamido)butyl)-N-(3-(2-chloroacetamido)propyl)acetamide) was synthesized following General procedure A using *N¹*-(3-aminopropyl)butane-1,4-diamine as the triamine component.

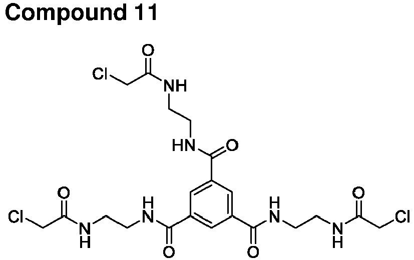

Compound **11** (*N¹*,*N³*,*N⁵*-tris(2-(2-chloroacetamido)ethyl)benzene-1,3,5-tricarboxamide) was synthesized following General procedure A using *N¹*,*N³*,*N⁵*-tris(2-aminoethyl)benzene-1,3,5-tricarboxamide as the triamine component.

### General procedure B for Finkelstein reactions

Trichloro acetamide (1 equiv.) was dissolved in acetone (550 uL/35 µmol) at 40 °C under stirring. After leaving the solution cool down to rt, NaI (9 equiv.) was added as a solution in acetone (1 M) and the mixture was stirred at 40 °C overnight. The reaction mixture was filtered over a pipette silica pad and washed with DCM/MeOH 9:1. Evaporation of the volatiles under reduced pressure afforded the triiodo acetamides in satisfying purity.

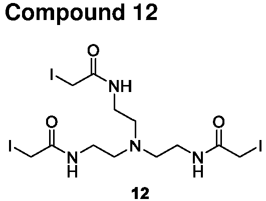

Compound **12** (*N*,*N*',*N*"-(Nitrilotris(ethane-2,1-diyl))tris(2-iodoacetamide)) was synthesized following General procedure B using compound **1** as the trichloro acetamide component.

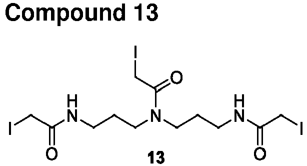

Compound **13** (2-Iodo-*N*,*N*-bis(3-(2-iodoacetamido)propyl)acetamide) was synthesized following General procedure B using compound **4** as the trichloro acetamide component.

### Synthesis of compound 14 (2-Bromo-N,N-bis(3-(2-bromoacetamido)propyl)acetamide)

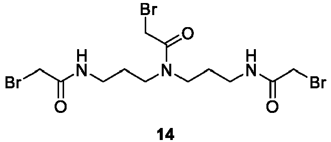

To a solution of dipropylenetriamine (1 equiv., 465 mg, 0.5 mL, 3.54 mmol) in DCM (3.54 mL) was added solid NaHCOs (3.2 equiv., 952.58 mg, 11.34 mmol) at rt. The mixture was cooled to 0 °C and bromoacetyl chloride (3.9 equiv., 2175.13 mg, 1.14 mL, 13.82 mmol) was added over 5 min. Next, water (7 mL) was added dropwise under vigorous stirring at 0 °C and the reaction mixture was allowed to warm to rt. After 6 h, the reaction mixture was diluted with 15 mL of water, the phases were separated, and the aq. phase was extracted with DCM (3 × 20 mL). The combined org. phases were washed with sat. brine (2 × 30 mL), dried over sodium sulfate and the volatiles were removed under reduced pressure to yield a colourless resin (415.2 mg). Purification of a small amount of crude via preparative HPLC H₂O + 0.05% TFA/MeCN + 0.1% FA, afforded compound **14** as a colourless solid.

### Synthesis of compound 15 (N,N',N"-(Nitrilotris(ethane-2,1-diyl))triacrylamide)

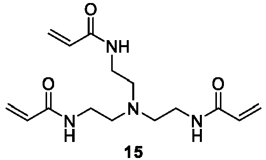

To a solution of *N¹*,*N¹*-bis(2-aminoethyl)ethane-1,2-diamine (1.0 equiv.) in DCM (1 M) was added K₂CO₃ (5.0 equiv., 6 M) in H₂O at 0 °C. Upon vigorous stirring, acryloyl chloride (3.3 equiv.) was added dropwise, and the mixture was allowed to stir at room temperature overnight. The mixture was diluted with water and extracted three times with DCM. Subsequently, the organic layer was washed with brine twice, dried over Na₂SO₄. MeOH was added to reach 10% and the solution was filtered through a short pad of silica and washed with MeOH/DCM 1:9. The filtrate was concentrated in vacuo to give a highly viscous yellow oil. A portion of the crude was further purified by flash chromatography DCM/MeOH to give compound **15** (*N*,*N'*,*N"*-(Nitrilotris(ethane-2,1-diyl))triacrylamide) as a white solid.

### Synthesis of compound 16 (N,N',N"-(Nitrilotris(ethane-2,1-diyl))tris(4-(cyanoethynyl)benzamide))

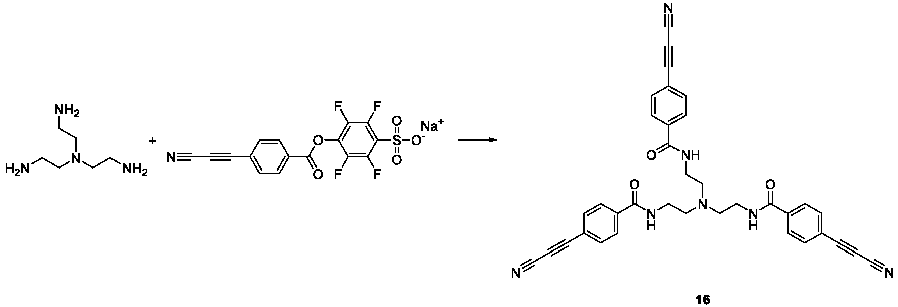

A solution of *N¹*,*N¹*-bis(2-aminoethyl)ethane-1,2-diamine (1 equiv., 50 µL, 0.005 mmol) in DMSO (0.1 M) was added to a solution of CBTF (Sodium 4-((4-(cyanoethynyl)benzoyl)oxy)-2,3,5,6-tetrafluorobenzenesulfonate) (3.3 equiv., 165 µL, 0.017 mmol) in DMSO (0.1 M) and the reaction was left for 3 h at rt. The crude product was purified via HPLC (H₂O/MeCN) to give compound **16** as a red solid.

### Synthesis of compound 17 (N,N',N"-(Nitrilotris(ethane-2,1-diyl))tris(1-(1-(4-(cyanoethynyl)phenyl)-1H-1,2,3-triazol-4-yl)-2,5,8,11,14-pentaoxaheptadecan-17-amide))

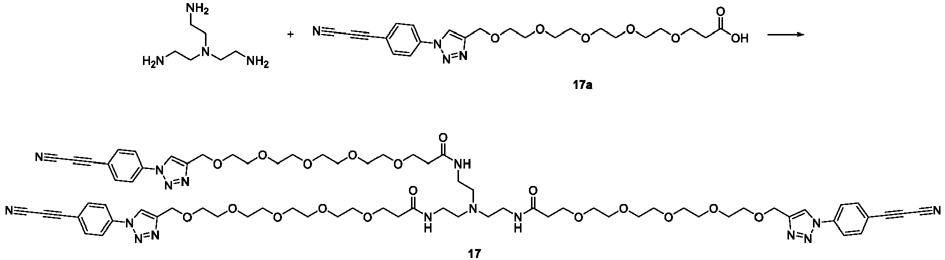

To the solution of acid **17a** (3 equiv., 9.7 mg, 0.0205 mmol) in DMF (0.55 mL) was added DIPEA (15 equiv., 13.27 mg, 0.018 mL, 0.103 mmol), then 2-bromo-1-ethyl-pyridinium tetrafluoroborate (BEP) (3.3 equiv., 6.18 mg, 0.023 mmol) as a solution in DMF (0.5 M). The resulting solution was left at room temperature for 5 minutes, then *N¹*,*N¹*-bis(2-aminoethyl)ethane-1,2-diamine (1 equiv., 0.068 mL, 0.0068 mmol) was added as a solution in DMSO (0.1 M) and the reaction was left for 15 min at rt. The crude product was purified via HPLC (H2O/MeCN) to give compound **17** as a red solid.

### Synthesis of compound 18 (2,4,6-Tris((4-fluorophenyl)thio)-1,3,5-triazine)

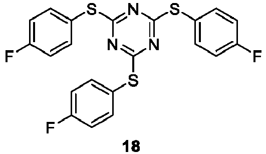

To K₂CO₃ (9 equiv., 2.99 mg, 0.022 mmol) was added cyanuric chloride (1 equiv., 80 µL, 0.0024 mmol) as a solution in acetone/dioxane (1:1) and then 4-fluorothiophenol (9 equiv., 62 µL, 0.022 mmol) as a solution in dioxane at rt. The mixture was stirred at 65 °C for 100 min and finally quenched with sat. aq. NH₄Cl until all solids were dissolved. The crude mixture was purified by HPLC (H₂O/MeCN) to give compound **18** as a white solid.

### Synthesis of compound 19 (2,4,6-Tris((4-chlorophenyl)thio)-1,3,5-triazine)

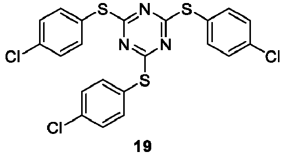

To K₂CO₃ (9 equiv., 2.99 mg, 0.022 mmol) was added cyanuric chloride (1 equiv., 80 µL, 0.0024 mmol) as a solution in acetone/dioxane (1:1) and then 4-chlorothiophenol (9 equiv., 154 µL, 21.6 µmol) as a solution in dioxane at rt. The mixture was stirred at 65 °C for 100 min and finally quenched with sat. aq. NH₄Cl until all solids were dissolved. The crude mixture was purified by HPLC (H₂O/MeCN) to give compound **19** as an off-white solid.

### Protein conjugation

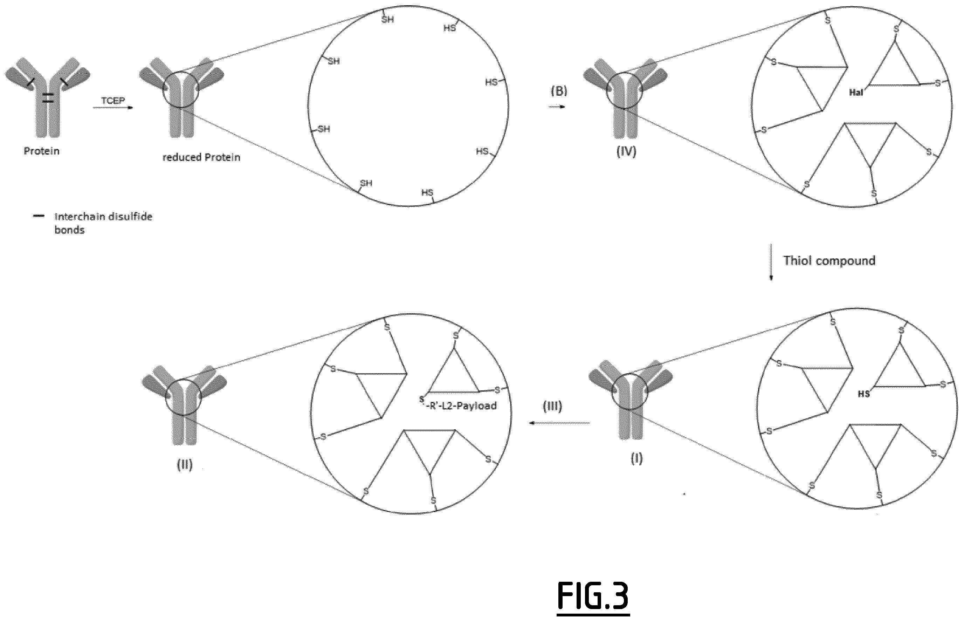

The general scheme of the protein conjugation is depicted in Figure 3.

### General procedure C for the preparation of conjugates (IV) by Reduction and rebridging

To a solution of Protein (50 µL, 50 µM in PBS pH 7.4) were added as follows EDTA (0.5 µL, 0.5 M in water, pH 8.0), TCEP·HCl (10 mM water, 2.0 µL, 20 nmol, 8.0 equiv.), and last the re-bridging agent (3 mM in HEPES pH 8.5, 50 mM HEPES, 50 mM NaCl, 26.25 nmol, 10.5 equiv.) resulting in a PBS/HEPES buffer mixture with pH 8.1. The mixture was vortexed upon addition of each component. The mixture was incubated at 37 °C for 4 hours on a thermal shaker at 500 rpm. The resulting conjugate (IV) was purified by Bio-Spin^{®} P-30Gel Columns (Bio-Rad) which had been equilibrated with PBS buffer (1X, pH 7.4).

### General procedure D for the preparation of conjugates (I)

To a solution of the conjugate (IV) (50 µL, 50 µM) in PBS buffer (1X, pH 7.4) was added a solution of thiol compound (24 mM, 240 equiv.) in HEPES buffer (pH 8.3, 100 mM HEPES) and the mixture was incubated at 25 °C for 18 hours on a thermal shaker at 500 rpm. The resulting conjugate (I) was purified by Bio-Spin^{®} P-30Gel Columns (Bio-Rad) which had been equilibrated with PBS buffer (1X, pH 7.4). The product was characterized by RP-HPLC-MS following General procedure G.

### General procedure E for the preparation of conjugates (II)

To a solution of the conjugate (I) (50 µL, 25 µM) in PBS buffer (1X, pH 7.4) was added a solution of thiol-reactive compound (III) (10 mM, 20 equiv.) in DMSO and the mixture was incubated until reaction completion (confirmed by HIC). The conjugate (II) was purified by Bio-Spin^{®} P-30Gel Columns (Bio-Rad) which had been equilibrated with PBS buffer (1X, pH 7.4). The product was characterized by HIC following General procedure F and by RP-HPLC-MS following General procedure G.

### General procedure F for hydrophobic interaction chromatography (HIC)

HIC was performed using a liquid chromatography system (Alliance HPLC, Waters, Manchester, UK) coupled to a 2487 UV/visible detector (Waters, Manchester, U.K.). Analyses were conducted on a TSKButyl-NPR, 3.5 × 4.6 mm column (Tosoh Bioscience, Tokyo, Japan) set at room temperature. The mobile phase A consisted of 1.5 M ammonium sulfate and 25 mM potassium phosphate at pH 7.0, while mobile phase B was a mixture of 25 mM potassium phosphate and 25% isopropanol at pH 7. Separation was achieved with a linear gradient of 0%-100% B over 12 min at a flow rate of 0.8 mL/min. Analyses were performed by injecting 20 µg of sample diluted to 1 mg/mL with mobile phase A and integrating the UV area at 210 nm for each species.

A chromatogram typical of the mixture of protein conjugates comprising at least 50% (in number) of protein conjugates of formula (II) of the invention as obtained is depicted in Figure 4.

### General procedure G for reverse-phase high performance liquid chromatography - mass spectrometry (RP-HPLC MS) characterization of the conjugates

RP-HPLC MS analyses were conducted using a ThermoFisher Ultimate 3000 HPLC system (Thermo Fisher Scientific, Germering, Germany), coupled to an Exactive^{™} Plus Extended Mass Range (EMR, Orbitrap analyzer, Thermo Fisher Scientific, Bremen, Germany). This setup was equipped with an HESI-II probe source operating in positive ion mode. Instrument control and data acquisition were handled using the Thermo Xcalibur^{™} mass spectrometry data system. For the analysis, an Agilent PLRP-S, 1000 Å, 2.1 × 50 mm, 5 µm column (P/N: PL1912-1502) was utilized and maintained at 40 °C. Mobile phase A was 0.1% formic acid in DI water, mobile phase B was 0.1% formic acid in acetonitrile. The separation process involved a flow rate of 0.600 mL/min and employed the following gradient: 0-4 min, isocratic 20% B; 4-5 min, 20% to 40% B; 5-10 min, 40% to 70% B; 10-11 min, 70% to 90% B. Full MS spectra were acquired in positive polarity with a scan range of 800-4,000 m/z. The resolution was set at 70,000, with an AGC target of 1 × 10⁶ ions. Sheath gas was set to 35 AU and auxiliary gas was set to 10 AU. The spray voltage was 2.2 kV, the capillary temperature was 250 °C. For each analysis, 1 µg of the protein conjugate was injected. All MS data of the conjugates were deconvoluted and analyzed using UniDec software (version 5.2.1, Marty et al. Anal. Chem. 2015. DOI: 10.1021/acs.analchem.5b00140).

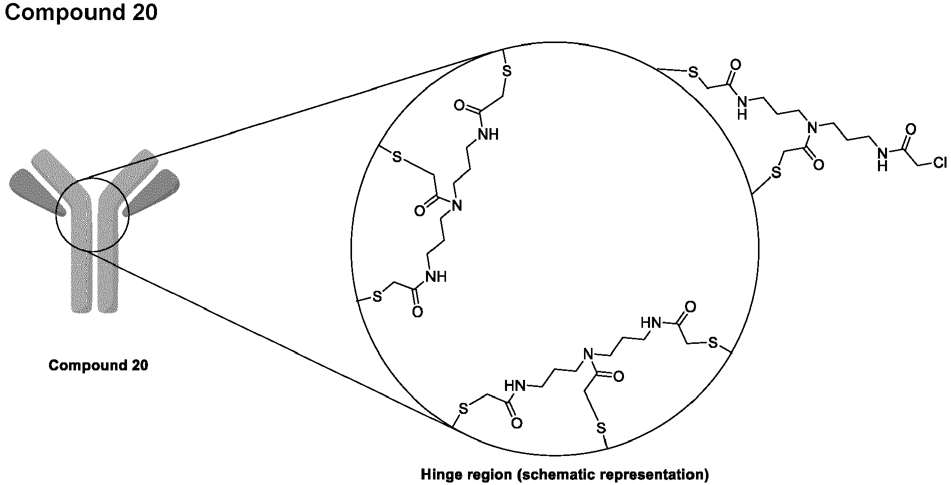

Compound 20 was prepared following General procedure C, using trastuzumab as the Protein and 2-chloro-*N*,*N*-bis(3-(2-chloroacetamido)propyl)acetamide (compound 4) as the re-bridging agent. RP-HPLC MS results: expected mass 146 656 Da; observed mass 146 664 Da.

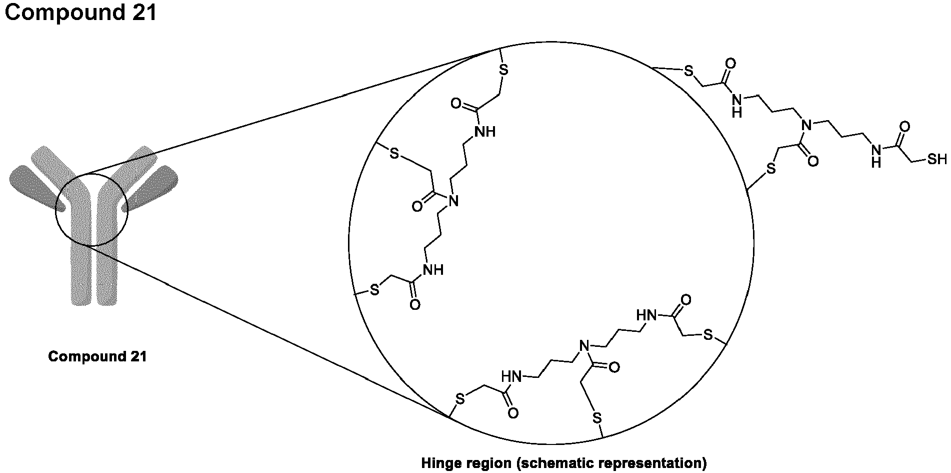

Compound **21** was prepared following General procedure D, using compound **20** as the conjugate (IV) and sodium hydrosulfide as the thiol compound. RP-HPLC MS results: expected mass 146 654 Da; observed mass 146 664 Da.

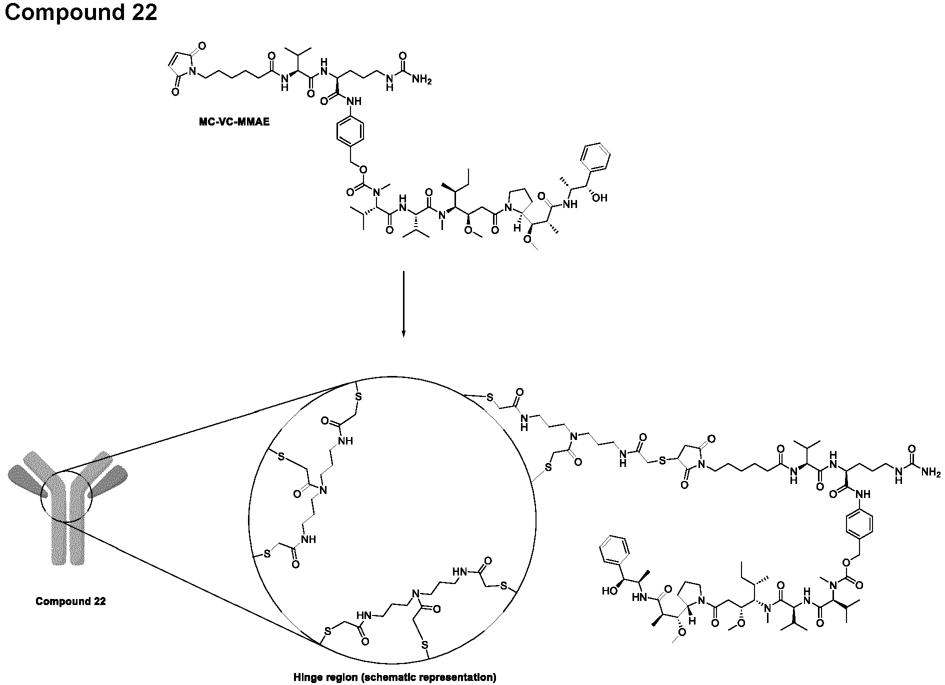

Compound **22** was prepared following General procedure E, using compound **21** as conjugate (II) and MC-VC-MMAE as thiol-reactive compound (III). The reaction mixture was incubated for a duration of 1.5 hours at 4 °C to ensure complete reaction. RP-HPLC MS results: expected mass 147 967 Da; observed mass 147 979 Da.

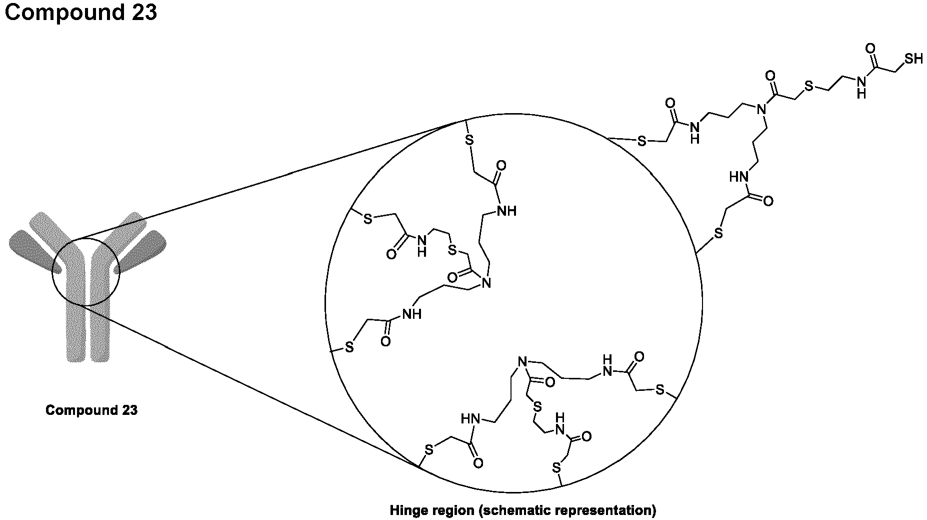

The precursor conjugate (IV) for compound **23** was prepared following General procedure C, using trastuzumab as the Protein and 2-((2-(2-chloroacetamido)ethyl)thio)-*N*,*N*-bis(3-(2-chloroacetamido)propyl)acetamide (compound **8**) as the re-bridging agent. Compound **23** was prepared following General procedure D, using the corresponding conjugate (IV) and sodium hydrosulfide as the thiol compound. RP-HPLC MS results: expected mass 147 011 Da; observed mass 147 015 Da.

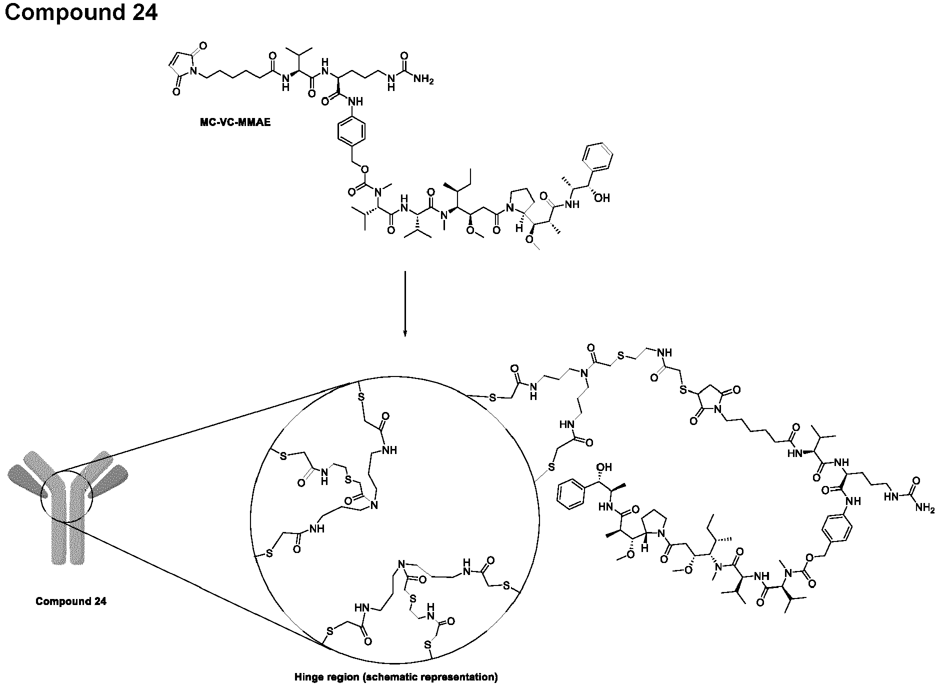

Compound **24** was prepared following General procedure E, using compound **23** as conjugate (II) and MC-VC-MMAE as thiol-reactive compound (III). The reaction mixture was incubated for a duration of 1.5 hours at 4 °C to ensure complete reaction. RP-HPLC MS results: expected mass 148 327 Da; observed mass 148 334 Da.

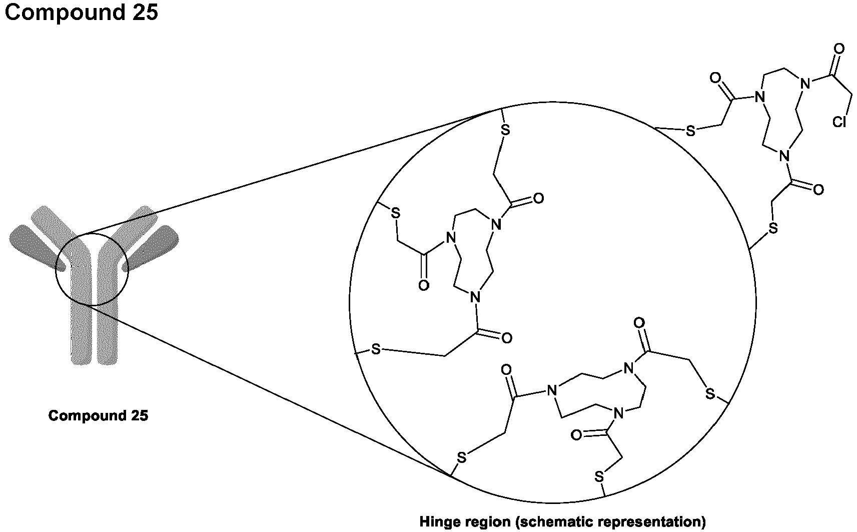

Compound **25** was prepared following General procedure C, using trastuzumab as the Protein and 1,1',1"-(1,4,7-triazonane-1,4,7-triyl)tris(2-chloroethan-1-one) (compound **5**) as the re-bridging agent. RP-HPLC MS results: expected mass 146 656 Da; observed mass 146 658 Da.

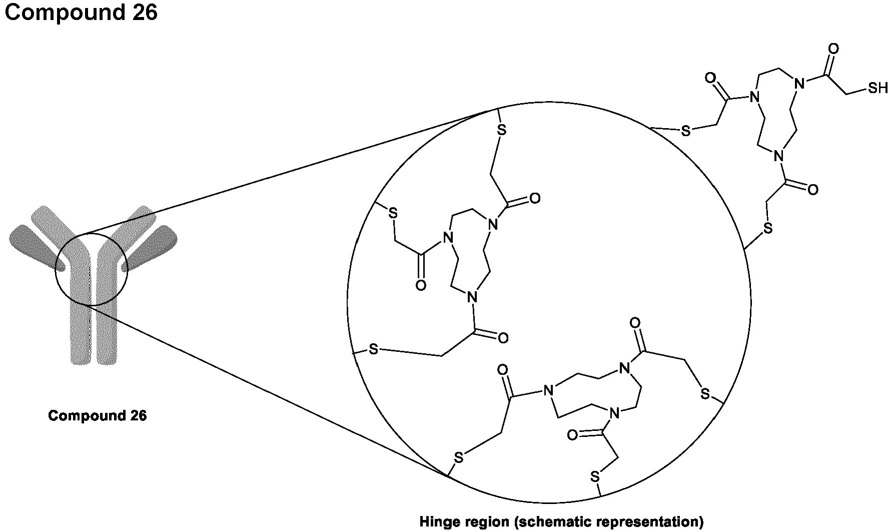

Compound **26** was prepared following General procedure D, using compound 25 as the conjugate (IV) and sodium hydrosulfide as the thiol compound. RP-HPLC MS results: expected mass 146 654Da; observed mass 146 657 Da.

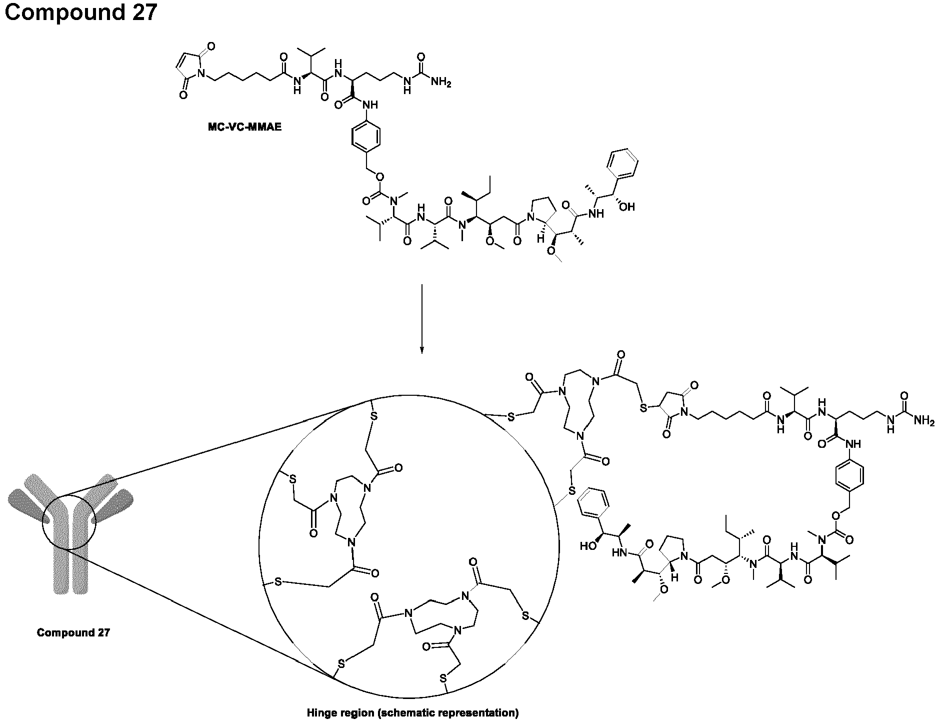

Compound **27** was prepared following General procedure E, using compound **26** as conjugate (II) and MC-VC-MMAE as thiol-reactive compound (III). The reaction mixture was incubated for a duration of 1.5 hours at 4 °C to ensure complete reaction. RP-HPLC MS results: expected mass 147 970 Da; observed mass 147 973 Da.

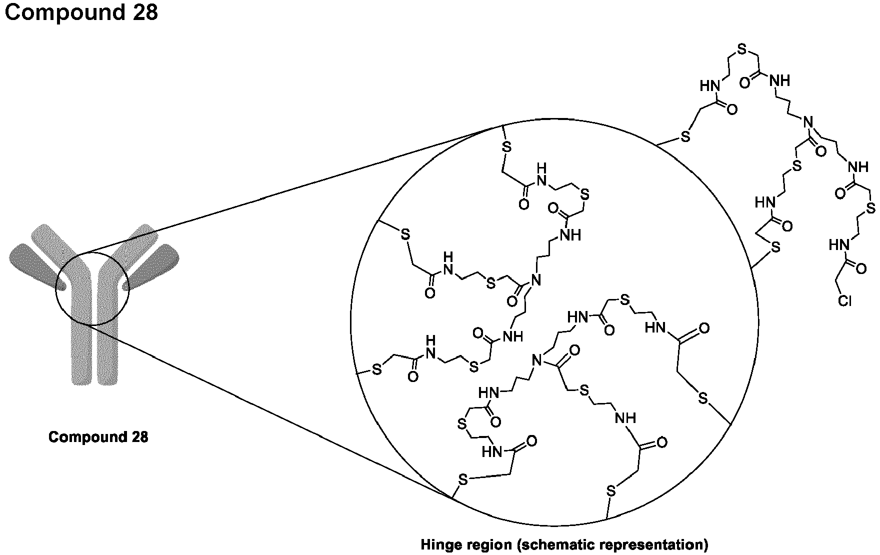

Compound **28** was prepared following General procedure C, using trastuzumab as the Protein and 2-((2-(2-chloroacetamido)ethyl)thio)-*N*,*N*-bis(3-(2-((2-(2-chloroacetamido)ethyl)thio)acetamido)propyl)acetamide (compound **9**) as the re-bridging agent. RP-HPLC MS results: expected mass 147 715 Da; observed mass 147 719 Da.

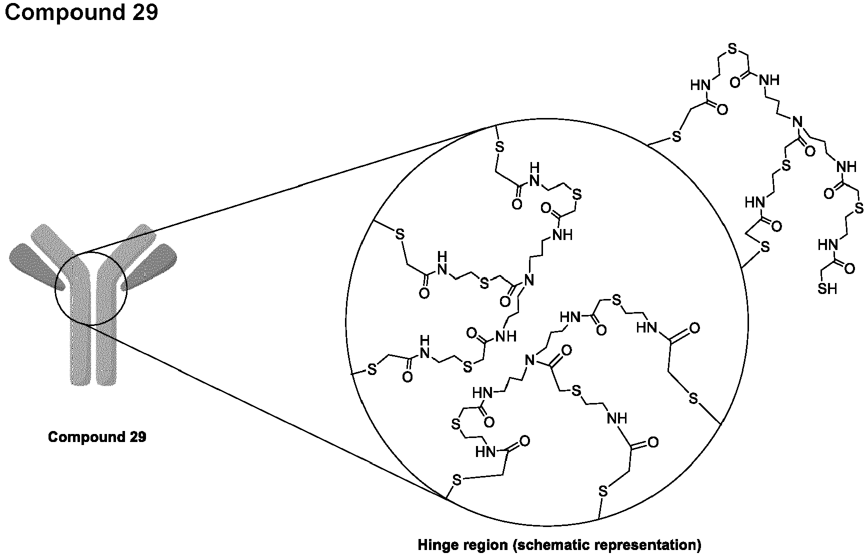

Compound **29** was prepared following General procedure D, using compound **28** as the conjugate (IV) and sodium hydrosulfide as the thiol compound. RP-HPLC MS results: expected mass 147 713 Da; observed mass 147 716 Da.

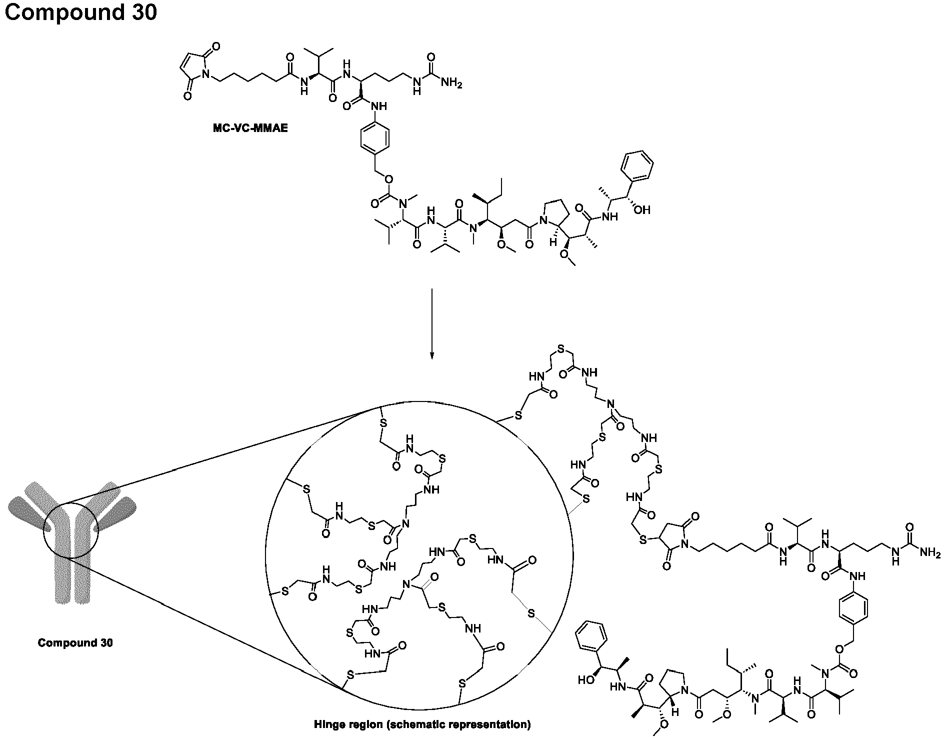

Compound **30** was prepared following General procedure E, using compound **29** as conjugate (II) and MC-VC-MMAE as thiol-reactive compound (III). The reaction mixture was incubated for a duration of 1.5 hours at 4 °C to ensure complete reaction. RP-HPLC MS results: expected mass 149 028 Da; observed mass 149 035 Da.

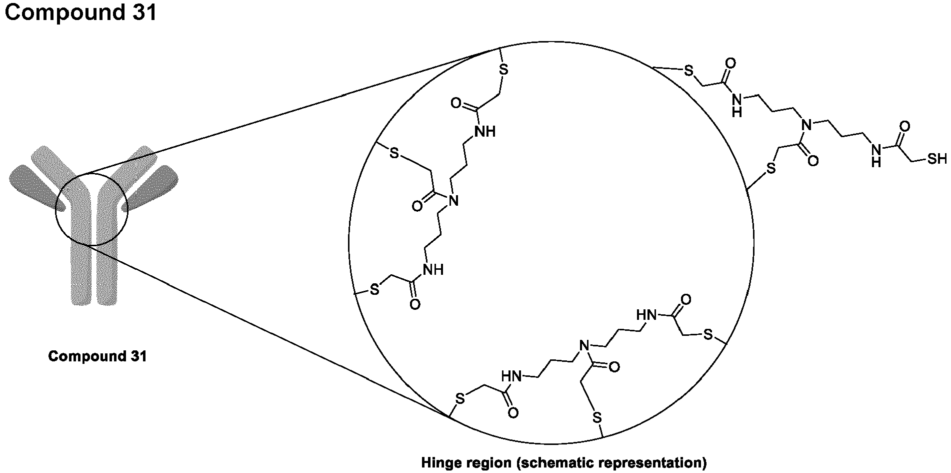

The precursor conjugate (IV) for compound **31** was prepared following General procedure C, using cetuximab as the Protein and 2-chloro-*N*,*N*-bis(3-(2-chloroacetamido)propyl)acetamide (compound **4**) as the re-bridging agent. Compound **31** was prepared following General procedure D, using the corresponding conjugate (IV) and sodium hydrosulfide as the thiol compound. RP-HPLC MS results: expected mass 151086 Da; observed mass 151 093 Da.

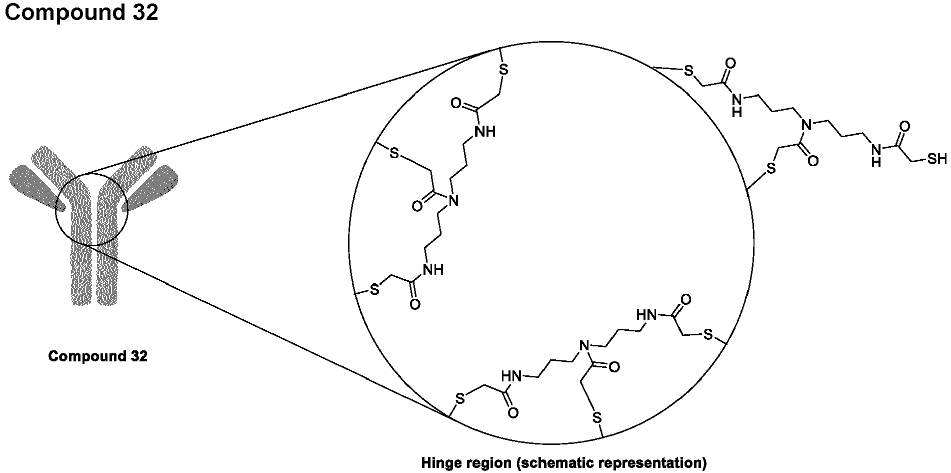

The precursor conjugate (IV) for compound **32** was prepared following General procedure C, using rituximab as the Protein and 2-chloro-*N*,*N*-bis(3-(2-chloroacetamido)propyl)acetamide (compound **4**) as the re-bridging agent. Compound **32** was prepared following General procedure D, using the corresponding conjugate (IV) and sodium hydrosulfide as the thiol compound. RP-HPLC MS results: expected mass 145 672 Da; observed mass 145 685 Da.

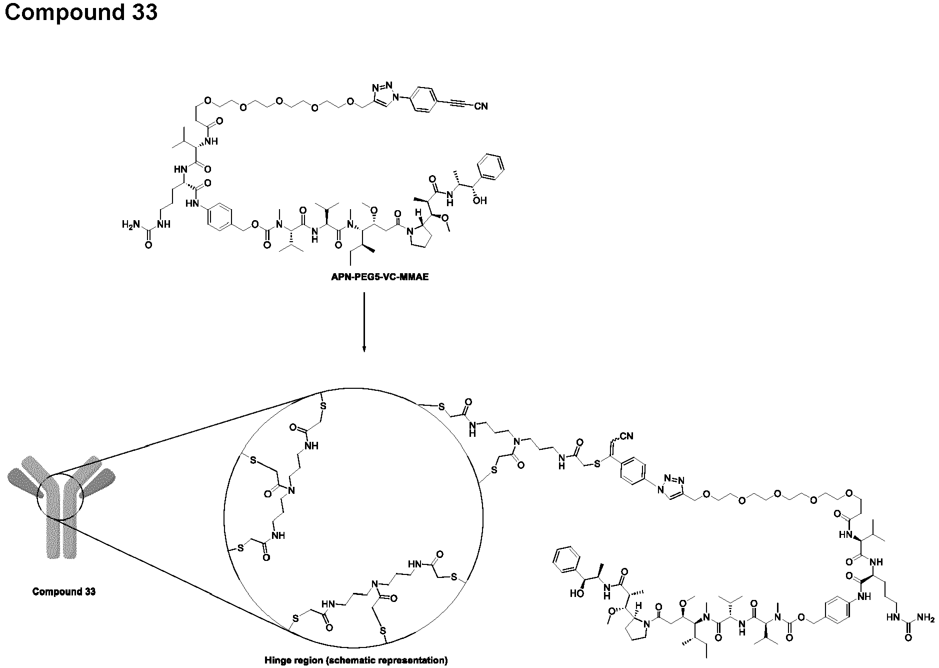

Compound **33** was prepared following General procedure E, using compound **32** as conjugate (II) and APN-PEG5-VC-MMAE as thiol-reactive compound (III). The reaction mixture was incubated for a duration of 18 hours at 25 °C to ensure complete reaction. RP-HPLC MS results: expected mass 147 252 Da; observed mass 147 262 Da.

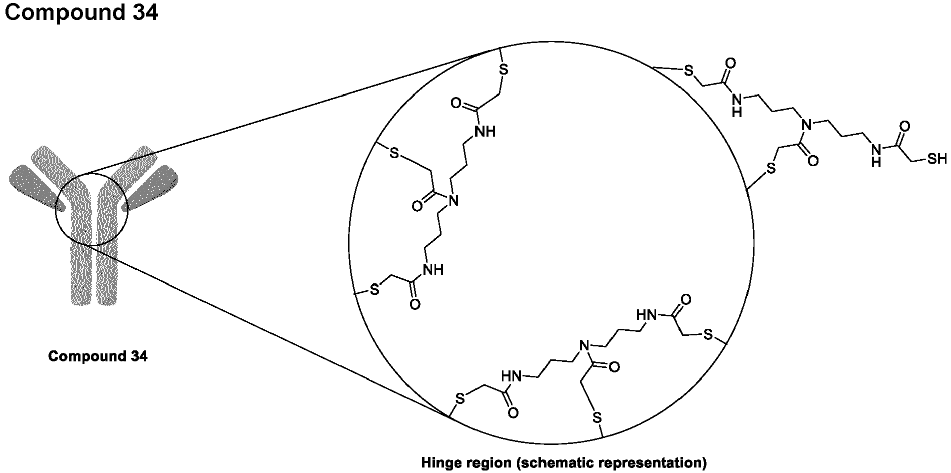

The precursor conjugate (IV) for compound **34** was prepared following General procedure C, using anti-human B7-H3 monoclonal antibody (Ombbio) as the Protein and 2-chloro-*N*,*N*-bis(3-(2-chloroacetamido)propyl)acetamide (compound **4**) as the re-bridging agent. Compound **34** was prepared following General procedure D, using the corresponding conjugate (IV) and sodium hydrosulfide as the thiol compound. RP-HPLC MS results: expected mass 146 359 Da; observed mass 146 367 Da.

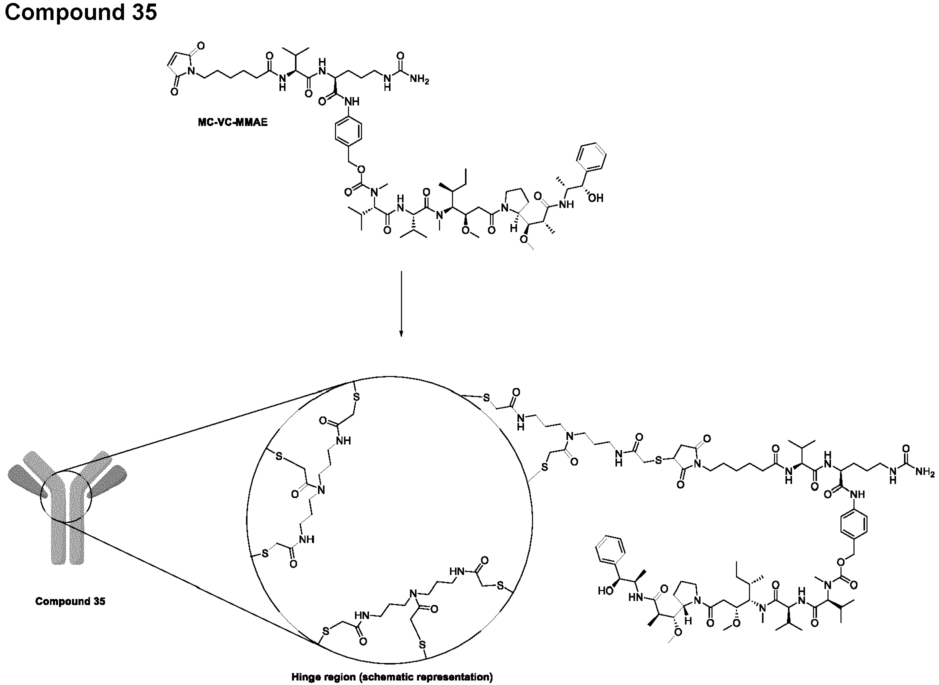

Compound **35** was prepared following General procedure E, using compound **34** as conjugate (II) and MC-VC-MMAE as thiol-reactive compound (III). The reaction mixture was incubated for a duration of 1.5 hours at 4 °C to ensure complete reaction. RP-HPLC MS results: expected mass 147 672 Da; observed mass 147 684 Da.

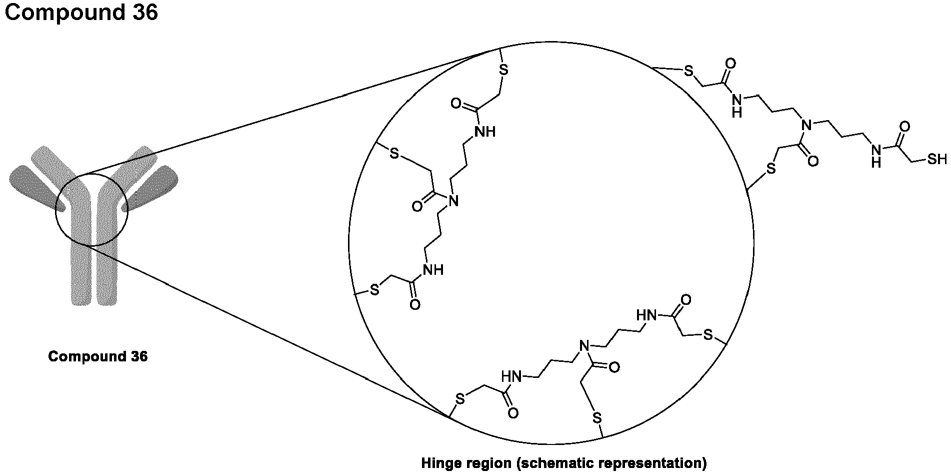

The precursor conjugate (IV) for compound **36** was prepared following General procedure C, anti-human Claudin 18.2 monoclonal antibody (Osebio) as the Protein and 2-chloro-*N*,*N-*bis(3-(2-chloroacetamido)propyl)acetamide (compound **4**) as the re-bridging agent. Compound **36** was prepared following General procedure D, using the corresponding conjugate (IV) and sodium hydrosulfide as the thiol compound. RP-HPLC MS results: expected mass 147 690 Da; observed mass 147 698 Da.

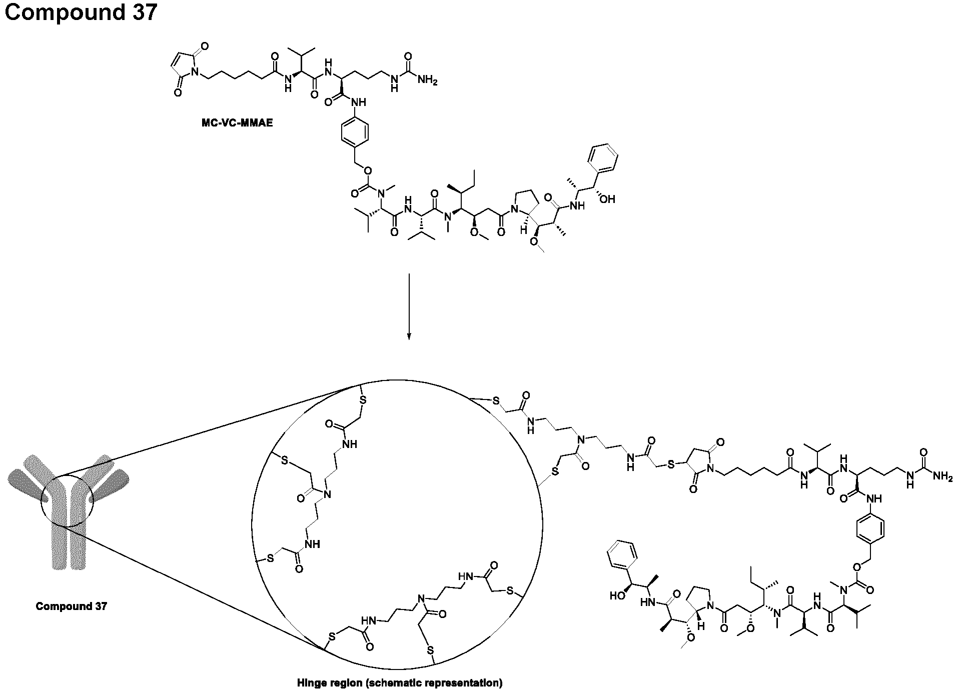

Compound **37** was prepared following General procedure E, using compound **36** as conjugate (II) and MC-VC-MMAE as thiol-reactive compound (III). The reaction mixture was incubated for a duration of 1.5 hours at 4 °C to ensure complete reaction. RP-HPLC MS results: expected mass 149 006 Da; observed mass 149 018 Da.

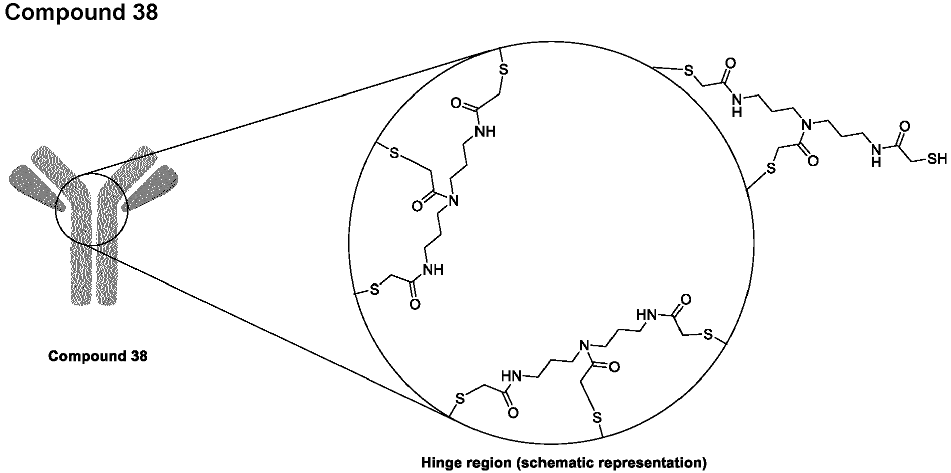

The precursor conjugate (IV) for compound **38** was prepared following General procedure C, anti-human Nectin-4 monoclonal antibody (Enfbio) as the Protein and 2-chloro-*N*,*N-*bis(3-(2-chloroacetamido)propyl)acetamide (compound **4**) as the re-bridging agent. Compound **38** was prepared following General procedure D, using the corresponding conjugate (IV) and sodium hydrosulfide as the thiol compound. RP-HPLC MS results: expected mass 145 263 Da; observed mass 145 271 Da.

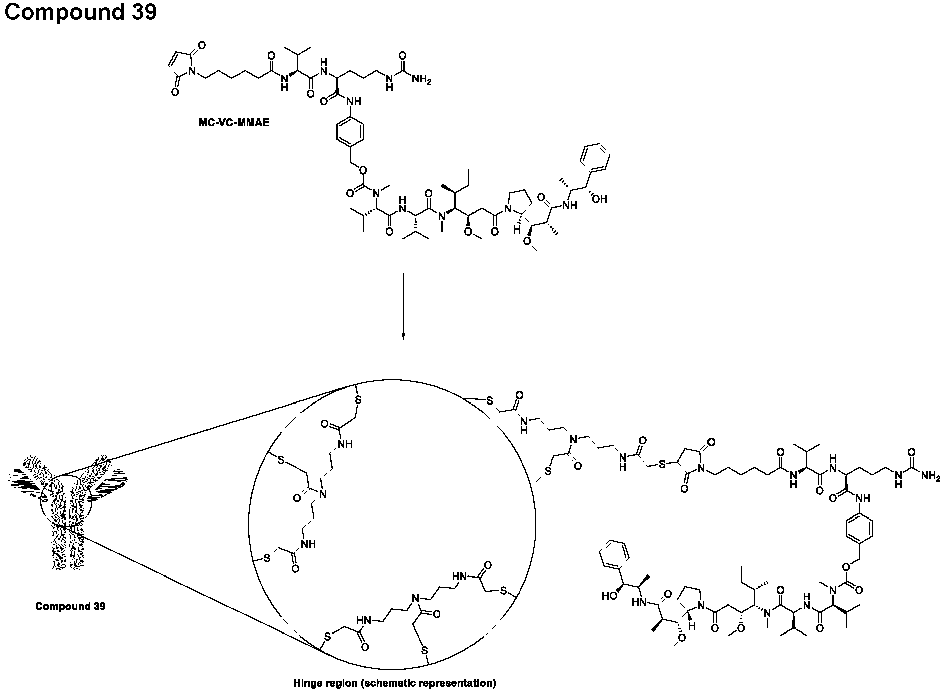

Compound **39** was prepared following General procedure E, using compound **38** as conjugate (II) and MC-VC-MMAE as thiol-reactive compound (III). The reaction mixture was incubated for a duration of 1.5 hours at 4 °C to ensure complete reaction. RP-HPLC MS results: expected mass 146 579 Da; observed mass 146 588 Da.

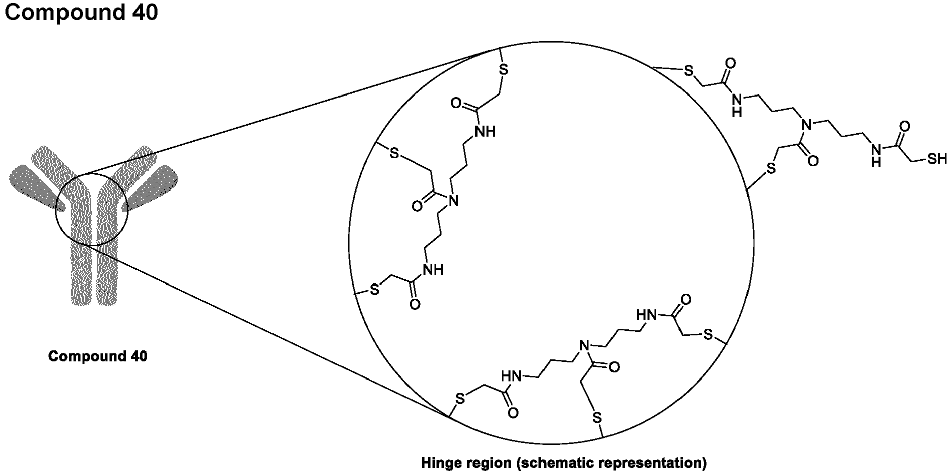

The precursor conjugate (IV) for compound **40** was prepared following General procedure C, anti-human TROP2 monoclonal antibody (Sacbio) as the Protein and 2-chloro-*N*,*N*-bis(3-(2-chloroacetamido)propyl)acetamide (compound **4**) as the re-bridging agent. Compound **40** was prepared following General procedure D, using the corresponding conjugate (IV) and sodium hydrosulfide as the thiol compound. RP-HPLC MS results: expected mass 146 608 Da; observed mass 146 615 Da.

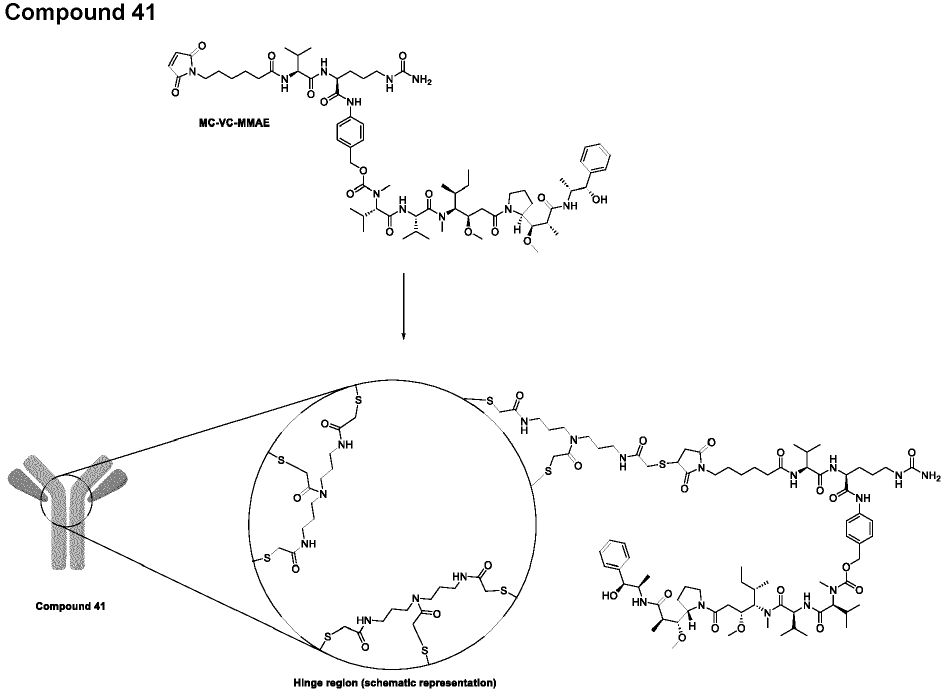

Compound **41** was prepared following General procedure E, using compound **40** as conjugate (II) and MC-VC-MMAE as thiol-reactive compound (III). The reaction mixture was incubated for a duration of 1.5 hours at 4 °C to ensure complete reaction. RP-HPLC MS results: expected mass 147 924 Da; observed mass 147930 Da.

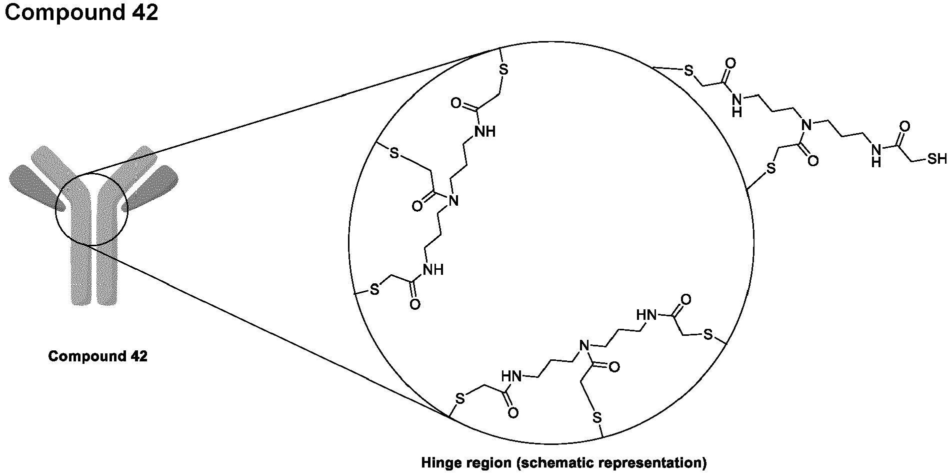

The precursor conjugate (IV) for compound **42** was prepared following General procedure C, anti-human ROR1 monoclonal antibody (Zilbio) as the Protein and 2-chloro-*N*,*N*-bis(3-(2-chloroacetamido)propyl)acetamide (compound **4**) as the re-bridging agent. Compound **42** was prepared following General procedure D, using the corresponding conjugate (IV) and sodium hydrosulfide as the thiol compound. RP-HPLC MS results: expected mass 146321 Da; observed mass 146321 Da.

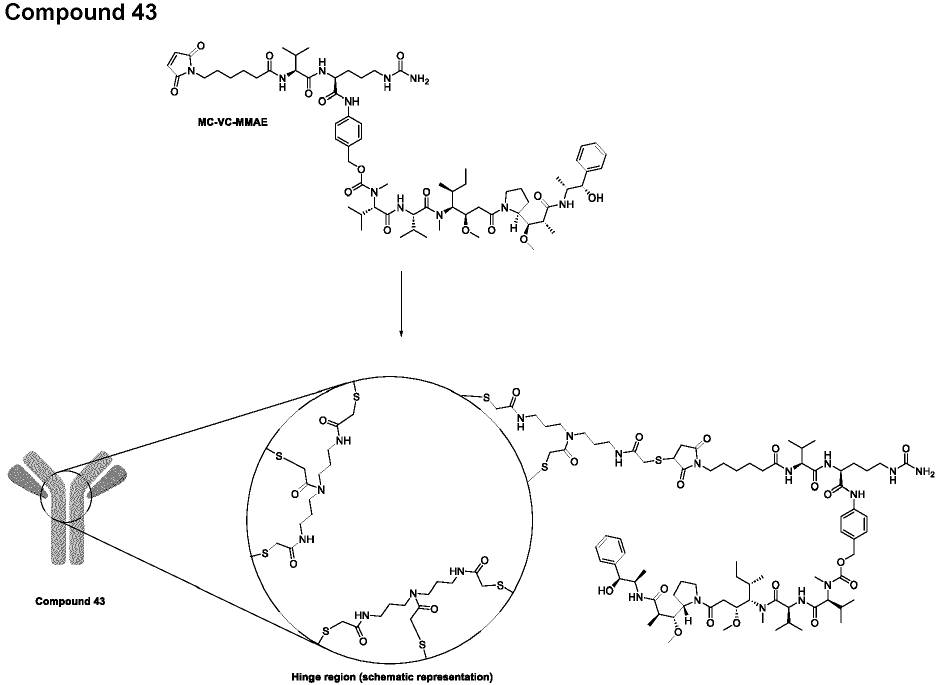

Compound **43** was prepared following General procedure E, using compound **42** as conjugate (II) and MC-VC-MMAE as thiol-reactive compound (III). The reaction mixture was incubated for a duration of 1.5 hours at 4 °C to ensure complete reaction. RP-HPLC MS results: expected mass 147 637 Da; observed mass 147 646 Da.

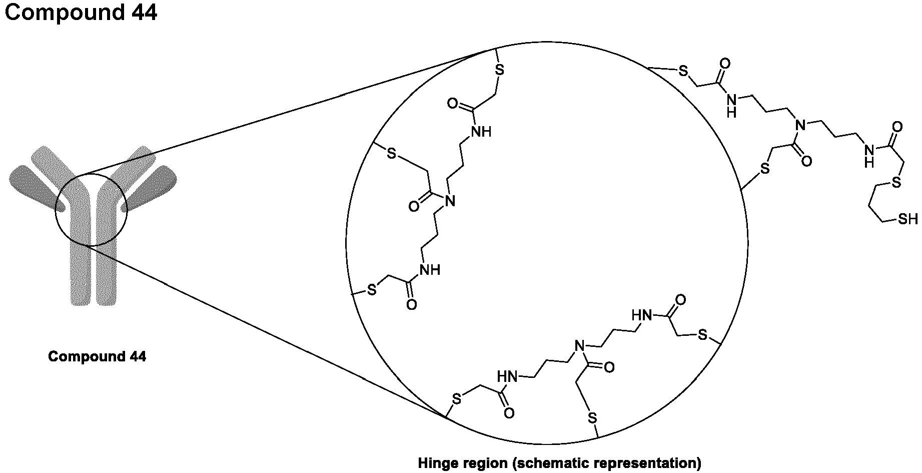

Compound **44** was prepared following General procedure D, using compound **20** as the conjugate (IV) and 1,3-propanedithiol as the thiol compound. RP-HPLC MS results: expected mass 146 728 Da; observed mass 146 739 Da.

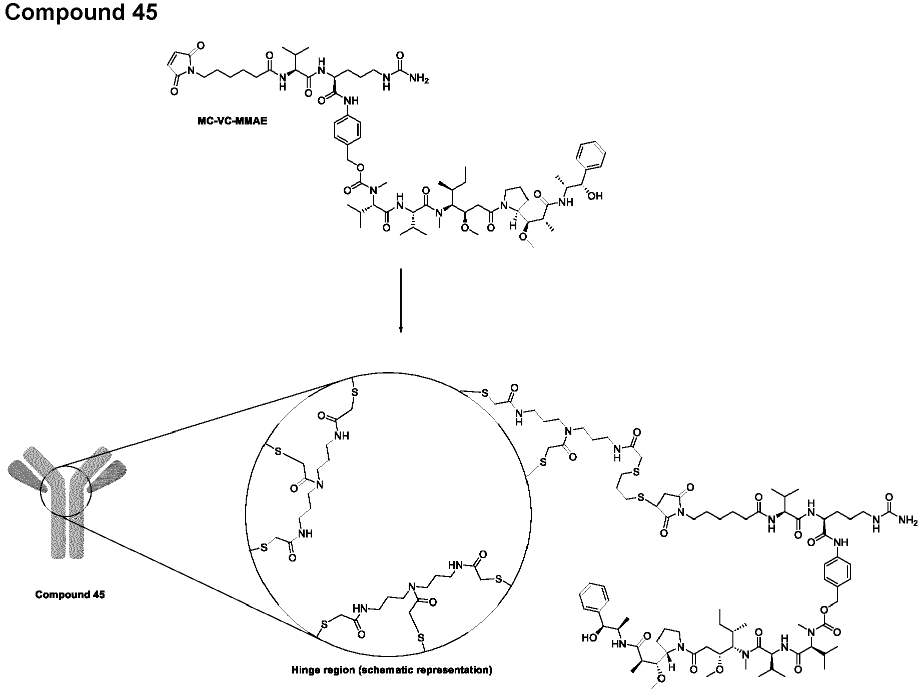

Compound **45** was prepared following General procedure E, using compound **44** as conjugate (II) and MC-VC-MMAE as thiol-reactive compound (III). The reaction mixture was incubated for a duration of 1.5 hours at 4 °C to ensure complete reaction. RP-HPLC MS results: expected mass 148 042 Da; observed mass 148 054 Da.

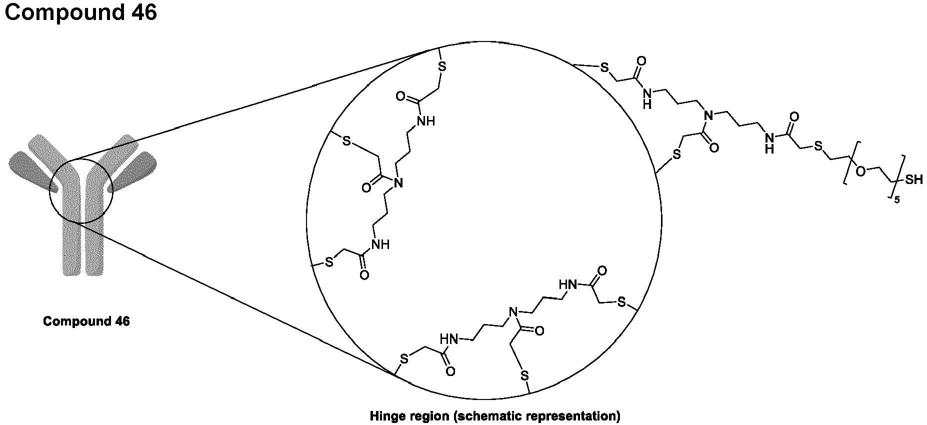

Compound **46** was prepared following General procedure D, using compound 20 as the conjugate (IV) and 3,6,9,12,15-pentaoxaheptadecane-1,17-dithiol as the thiol compound. RP-HPLC MS results: expected mass 146 934 Da; observed mass 146 943 Da.

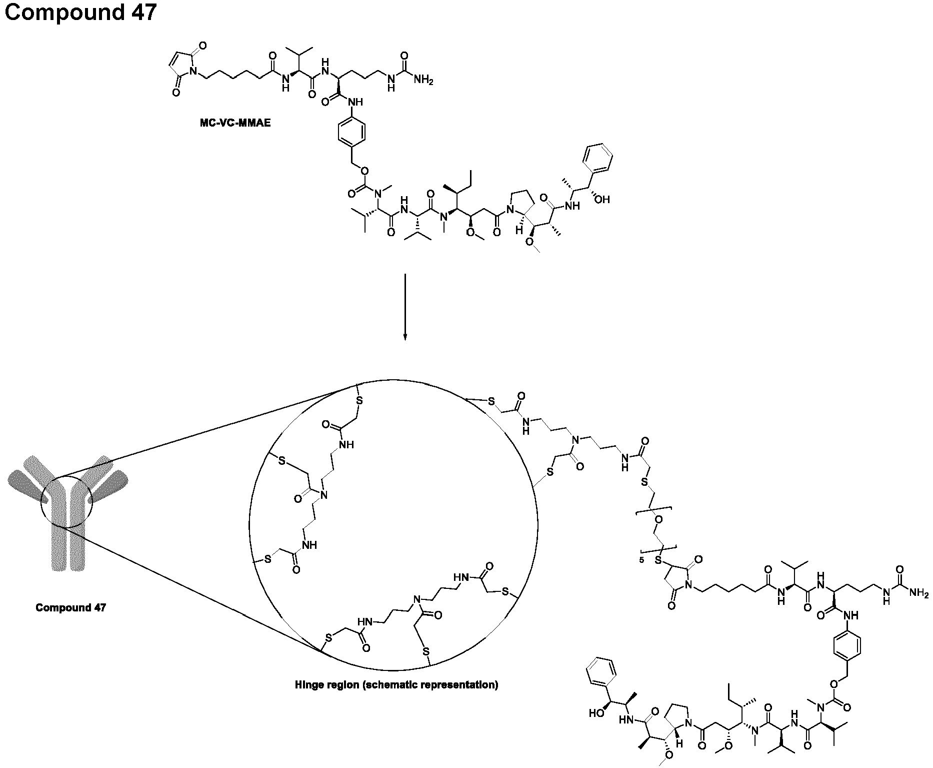

Compound **47** was prepared following General procedure E, using compound **46** as conjugate (II) and MC-VC-MMAE as thiol-reactive compound (III). The reaction mixture was incubated for a duration of 1.5 hours at 4 °C to ensure complete reaction. RP-HPLC MS results: expected mass 148 250 Da; observed mass 148 260 Da.

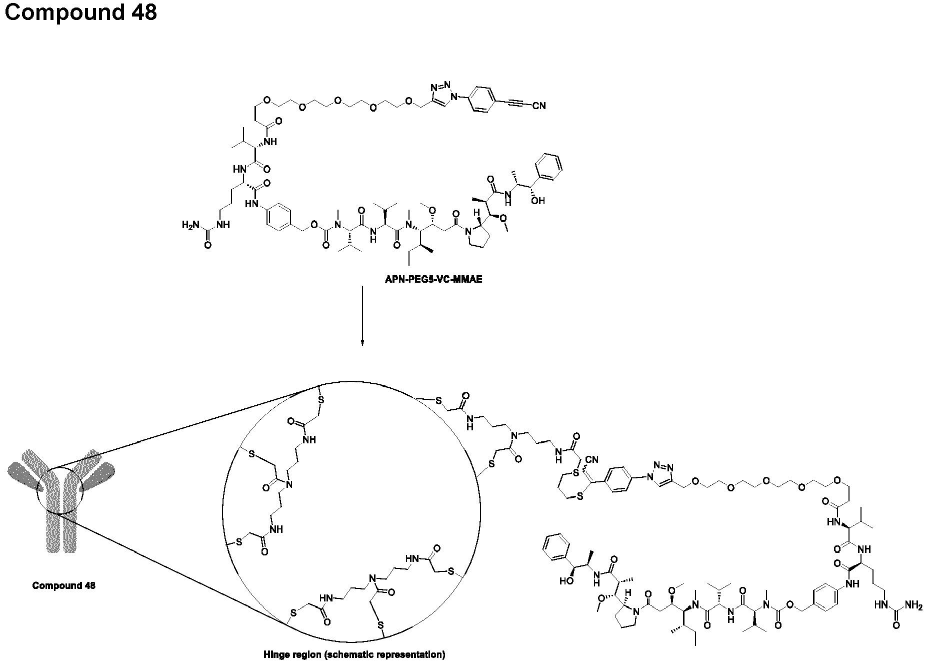

Compound **48** was prepared following General procedure E, using compound **44** as conjugate (II) and APN-PEG5-VC-MMAE as thiol-reactive compound (III). The reaction mixture was incubated for a duration of 18 hours at 25 °C to ensure complete reaction. RP-HPLC MS results: expected mass 148 303 Da; observed mass 148 317 Da.

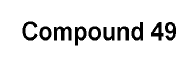

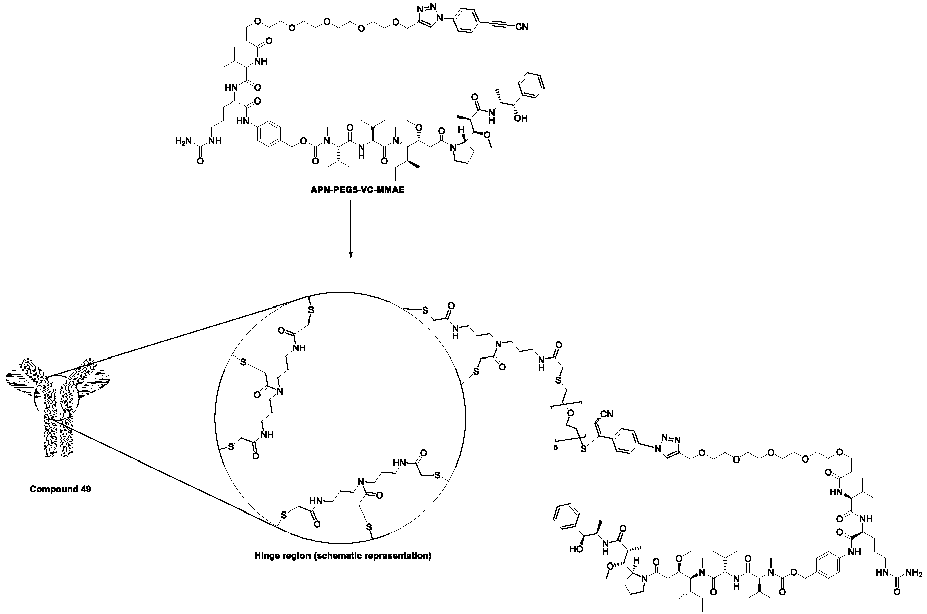

Compound **49** was prepared following General procedure E, using compound **46** as conjugate (II) and APN-PEG5-VC-MMAE as thiol-reactive compound (III). The reaction mixture was incubated for a duration of 18 hours at 25 °C to ensure complete reaction. RP-HPLC MS results: expected mass 148 511 Da; observed mass 148 521 Da.

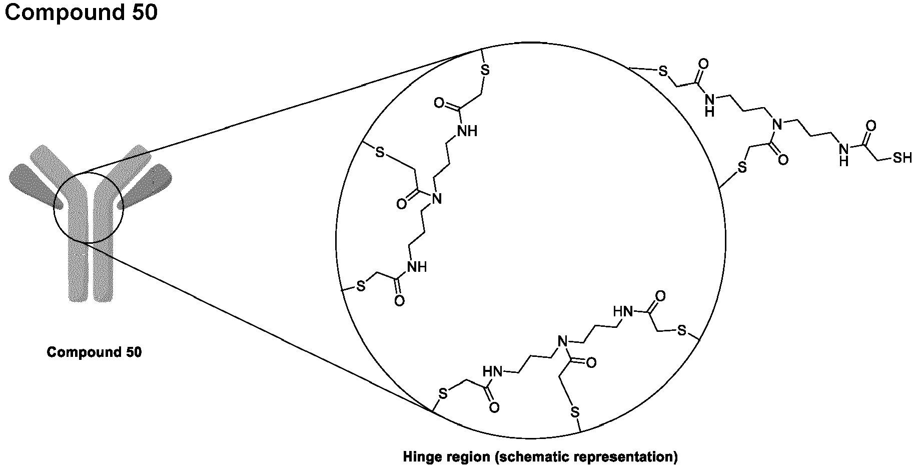

The precursor conjugate (IV) for compound **50** was prepared following General procedure C, J591 as the Protein and 2-chloro-*N*,*N*-bis(3-(2-chloroacetamido)propyl)acetamide (compound **4**) as the re-bridging agent. Compound **50** was prepared following General procedure D, using the corresponding conjugate (IV) and sodium hydrosulfide as the thiol compound. RP-HPLC MS results: expected mass 145 597 Da; observed mass 145 609 Da.

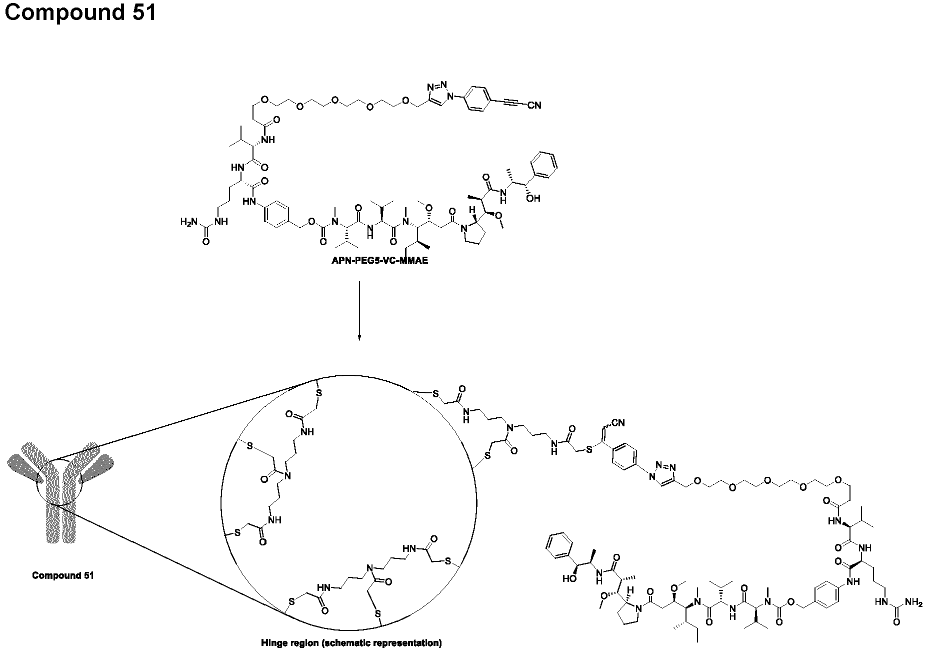

Compound **51** was prepared following General procedure E, using compound **50** as conjugate (II) and APN-PEG5-VC-MMAE as thiol-reactive compound (III). The reaction mixture was incubated for a duration of 18 hours at 25 °C to ensure complete reaction. RP-HPLC MS results: expected mass 147 175 Da; observed mass 147185 Da.

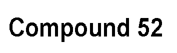

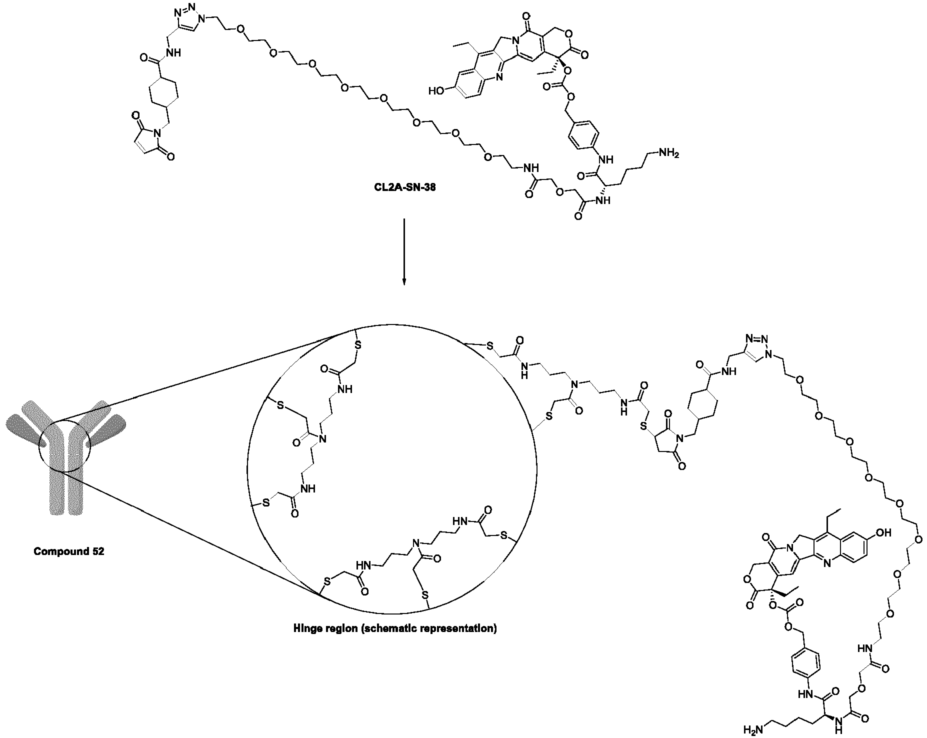

Compound **52** was prepared following General procedure E, using compound **21** as conjugate (II) and CL2A-SN-38 as thiol-reactive compound (III). The reaction mixture was incubated for a duration of 1.5 hours at 4 °C to ensure complete reaction. RP-HPLC MS results: expected mass 148 131 Da; observed mass 148 144 Da.

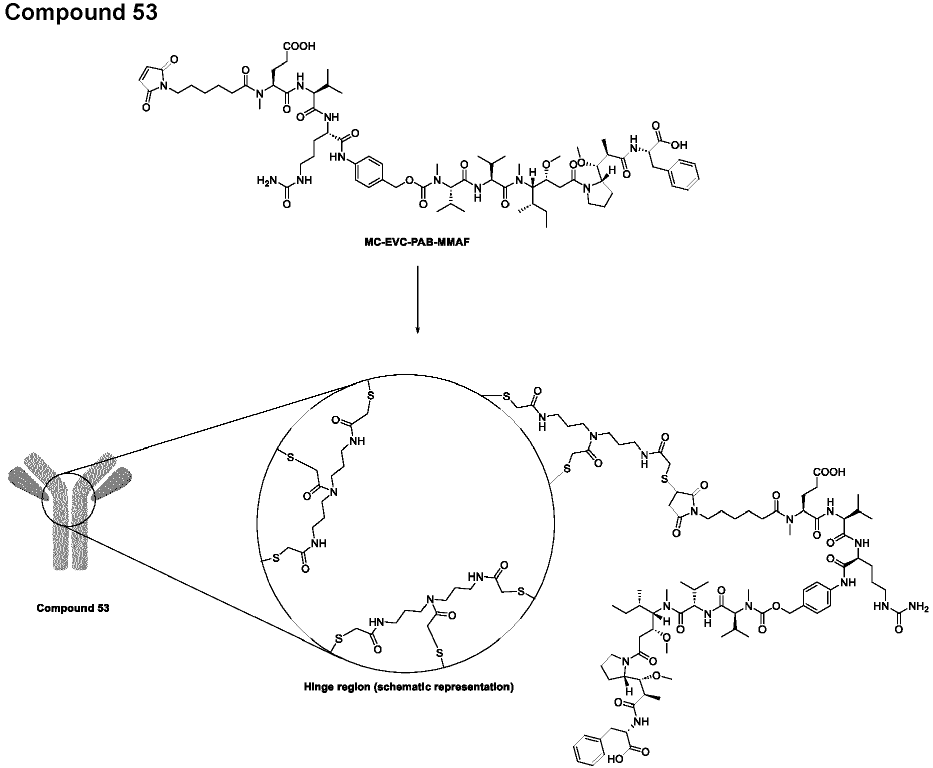

Compound **53** was prepared following General procedure E, using compound **21** as conjugate (II) and MC-EVC-PAB-MMAF as thiol-reactive compound (III). The reaction mixture was incubated for a duration of 1.5 hours at 4 °C to ensure complete reaction. RP-HPLC MS results: expected mass 148 124 Da; observed mass 148 124 Da.

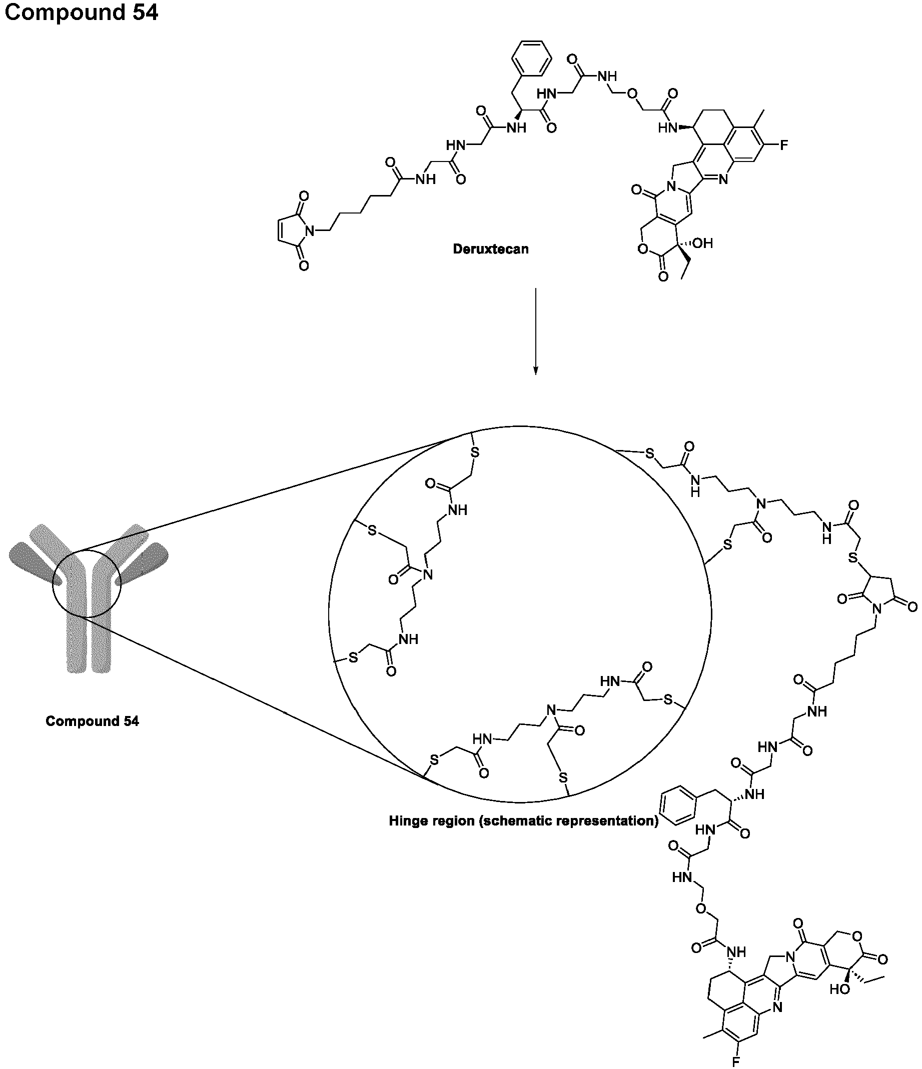

Compound **54** was prepared following General procedure E, using compound **21** as conjugate (II) and deruxtecan as thiol-reactive compound (III). The reaction mixture was incubated for a duration of 1.5 hours at 4 °C to ensure complete reaction. RP-HPLC MS results: expected mass 147 685 Da; observed mass 147 699 Da.

## Claims

1. A protein conjugate of formula (I):
Protein'-(L1)ₘ-SH (I)
Wherein
Protein' is a re-bridged protein or fragment thereof, comprising interchain cysteine residues, whereas at least two of said interchain cysteines are bridged together through the sulfur (S) atoms of said cysteine residues by a group of formula (A-1) and optionally, the remaining interchain cysteine residues are bridged three by three by one or more groups of formula (A-2): 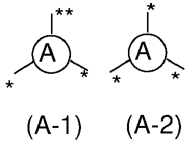 Where
the (A-1) group bridges together the -(L1)ₘ-SH fragment and two sulfur atoms of said cysteine residues;
the optional (A-2) group(s) bridge together three sulfur atoms of said cysteine residues;
A represents a tri-valent group,
each * designates a bond linking the A group of (A-1) and (A-2) with a sulfur atom of said cysteine residues;
** designates a bond linking the A group of (A-1) with the (L1)ₘ-SH fragment of conjugate (I);
SH is a thiol group;
L1 is an optional divalent group linking SH to the (A-1) group of Protein', said L1 preferably consisting of one or multiple successive units selected from the group consisting of : a linear or branched, saturated or unsaturated, C1-C60 alkylene group optionally interrupted and/or terminated on one or both sides by one or more chemical groups selected from -O-, -S-, - S(O)-, -SO₂-, -O-P(O)(OH)-O-, -C(O)-, -NH-, -NMe-, -C(O)NH-, -NHC(O)-, -NH-C(O)-NH, - O-C(O)-NH-, -O-C(O)-O-; C3-C8 cycloalkylene; C3-C8 heterocyclylene; 5 to 12 membered heteroarylene; C6-C12 arylene; amino acids; polypeptides; glycosylene; -(CH₂-CH₂-O-)ᵣ- groups; in which r is an integer ranging from 1 to 24 and/or optionally substituted by one or more of OH, CN, NH₂, CF₃, NHCONH₂, CONH₂, COOH, =O, =NH, glycosyl, C1-C12 alkyl, C6-C12 aryl, C5-C12 heteroaryl;
m is 0 or 1.

2. The protein conjugate according to claim 1 wherein Protein' derives from naturally occurring antibody having two heavy chains and two light chains, in which the sulfur atoms of said interchain cysteine residues form interchain disulfide bridges, the two heavy chains being bridged together by two disulfide bridges, and each heavy chain being bridged to a light chain by a disulfide bridge,
and Protein' differs from said naturally occurring antibody in that in Protein' the heavy and light chains are re-bridged through one (A-1) and two (A-2) groups.

3. The protein conjugate according to claim 1 wherein Protein' derives from an engineered antibody having two heavy chains and two light chains in which 6 out of 8 cysteines forming interchain disulfide bonds were mutated into any other amino acid, either the same or different, and wherein Protein' differs from said engineered antibody in that in Protein' the two heavy chains are re-bridged together through one (A-1) group, or two cysteines on the same heavy chain are rebridged.

4. The protein conjugate according to claim 1 wherein Protein' derives from an engineered antibody having two heavy chains and two light chains in which two cysteine groups have been introduced into the protein sequence, and wherein protein' differs from said engineered antibody in that in Protein' the two introduced cysteine groups are bridged through one (A-1) group.

5. The protein conjugate according to anyone of the preceding claims, wherein A is chosen from linear, branched or cyclic trivalent moieties, comprising 1 to 9 nitrogen atoms and from 0 to 30 carbon atoms, said moiety comprising three terminal functions of formula:
-NH-(C=O)-CH₂-
Where the terminal -CH₂- group is attached to the * or ** bond
Where * and ** are defined as in formula (A-1) and (A-2).

6. The protein conjugate according to anyone of the preceding claims where A is chosen from the group consisting in: 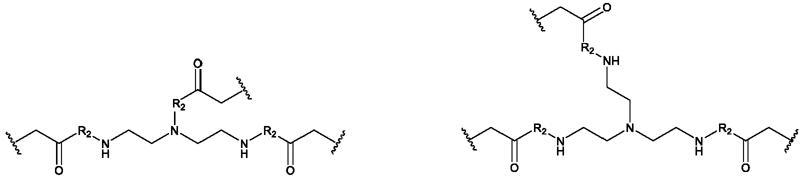 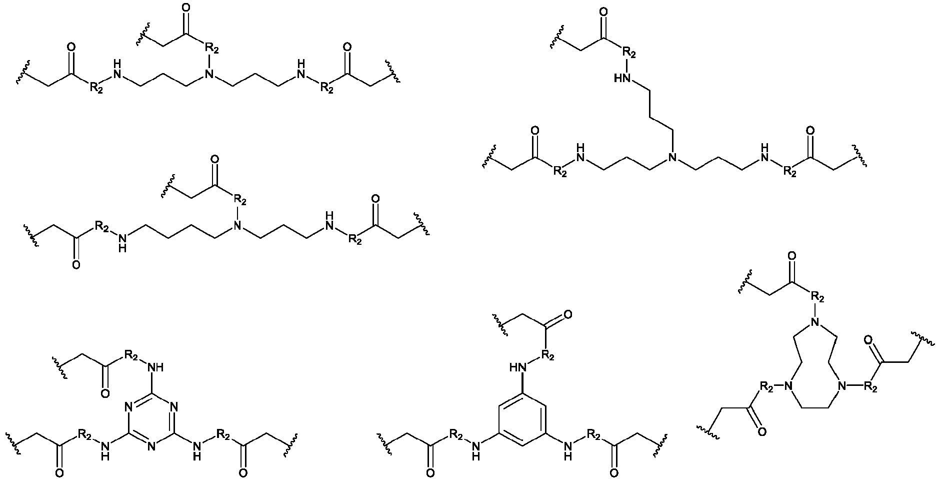 where R₂ is an optional spacer unit selected from the group consisting of a C6-C12 arylene; a linear or branched, saturated or unsaturated C1-C60 alkylene group optionally interrupted by one or more chemical groups selected from -O-, -S-, -S(O)-, -SO₂-, -O-P(O)(OH)-O-, - C(O)-, -NH-, -C(O)NH-, -NHC(O)-, -C(O)NR-, -NRC(O)-, heteroarylene, arylene, glycosyl, an -O-(CH₂-CH₂-O-)ᵣ -, -NH-(CH₂-CH₂-O-)ᵣ- , -(-O-CH₂-CH₂)ᵣ , -(CH₂-CH₂-O-)ᵣ- groups, amino acids and peptide residues, where R is H or a C1-C6 alkyl and r is an integer ranging from 1 to 24.

7. The protein conjugate according to anyone claims 1 to 6 where A is chosen from the group consisting in:

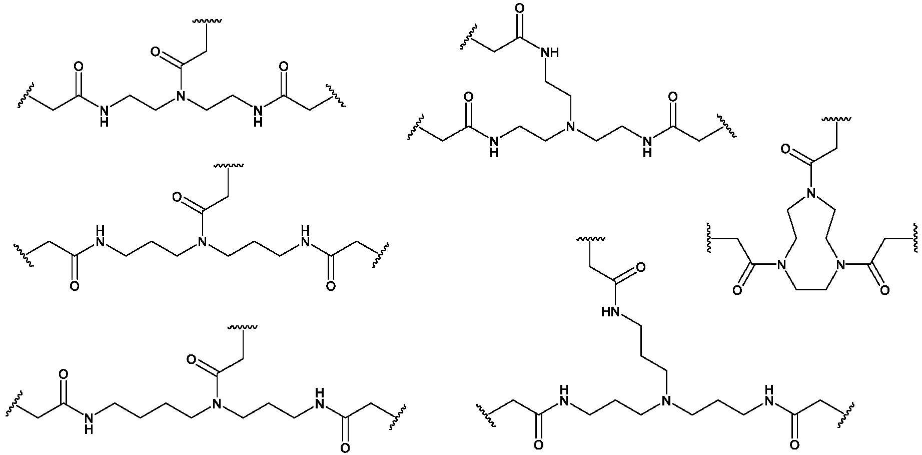

8. A protein conjugate of formula (II):
Protein'-(L1)ₘ-S-R'-L2-Payload (II)
Wherein
Protein', L1 and m are defined as in anyone of the preceding claims,
R' is a group formed as a result of a reaction between a thiol and a thiol-reactive group, such as maleimide, 3-arylpropiolonitrile, bromo-maleimide, chloroacetamide, bromoacetamide, iodoacetamide, acrylamide, vinyl sulfone, pyridyl disulfide, alpha-halo carbonyl, vinyl pyridine;
L2 is a divalent group connecting together the R' and Payload moieties, said L2 preferably consisting of one or multiple successive units selected from the group consisting of a linear or branched, saturated or unsaturated, C1-C60 alkylene group optionally interrupted and/or terminated on one or both sides by one or more chemical groups selected from -O-, -S-, - S(O)-, -SO₂-, -O-P(O)(OH)-O-, -C(O)-, -NH-, -NMe-, -C(O)NH-, -NHC(O)-, -NH-C(O)-NH, - O-C(O)-NH-, -O-C(O)-O-; C3-C8 cycloalkylene; C3-C8 heterocyclylene; 5 to 12 membered heteroarylene; C6-C12 arylene; amino acids; polypeptides; glycosylene; -(CH₂-CH₂-O-)ᵣ- groups; in which r is an integer ranging from 1 to 24 and/or optionally substituted by one or more of OH, CN, NH₂, CF₃, NHCONH₂, CONH₂, COOH, =O, =NH, glycosyl, C1-C12 alkyl, C6-C12 aryl, C5-C12 heteroaryl;
Payload is a molecule which exerts biological activity.

9. The protein conjugate according to claim 8 where said Payload is chosen from drug or imaging agents, oligonucleotides, chelating ligands capable of complexing with radionuclides, cytotoxic drugs and antineoplastic agents.

10. The protein conjugate according to anyone of claims 8 to 9 where -S-R'-L2-Payload is selected from the following structures:

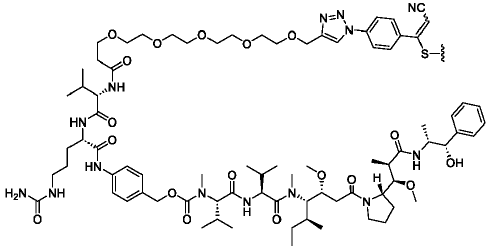

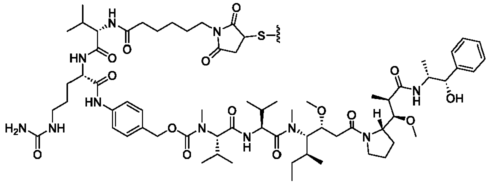

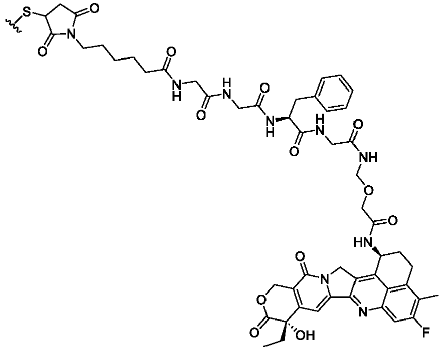

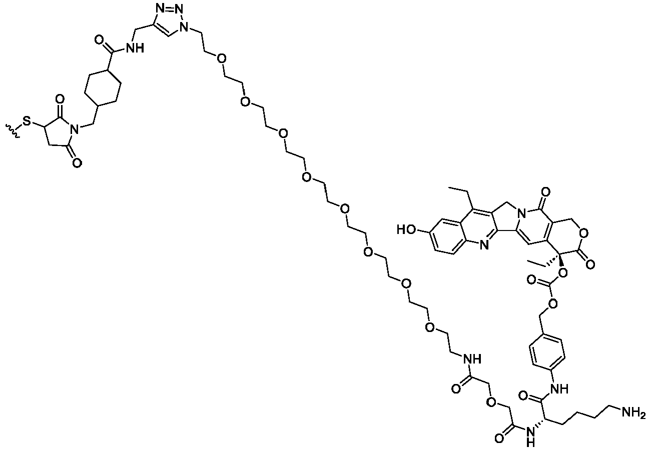

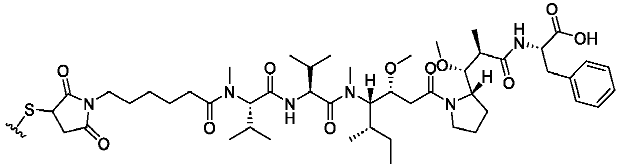

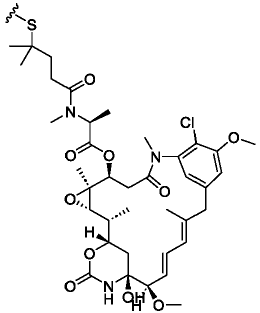

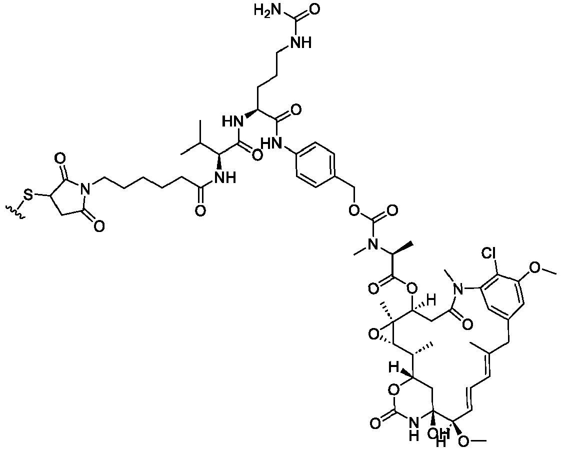

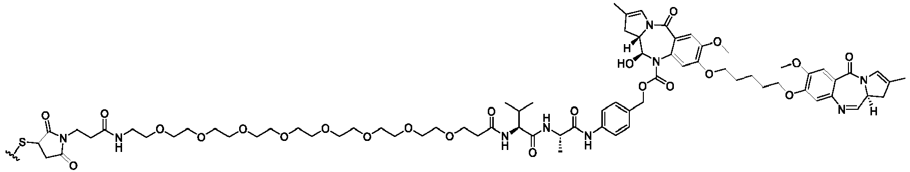

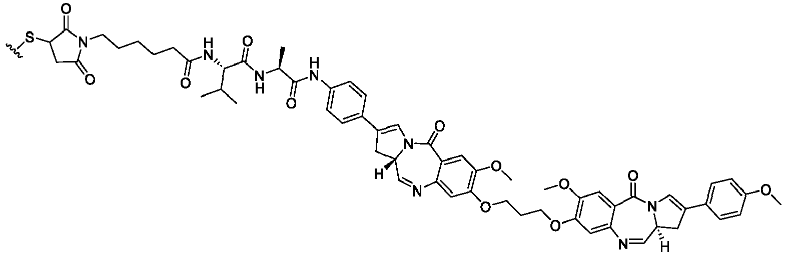

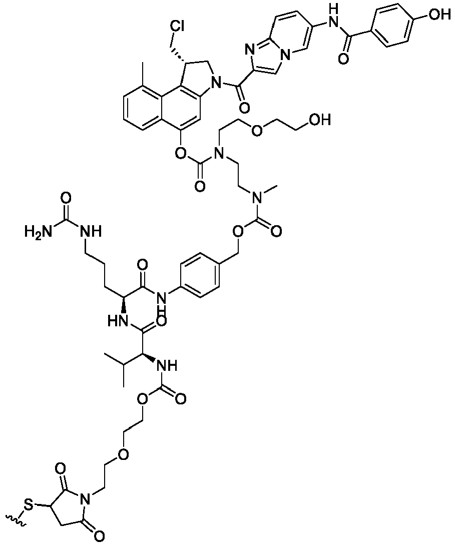

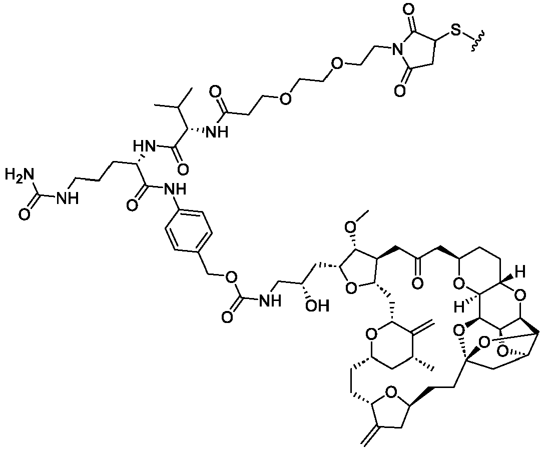

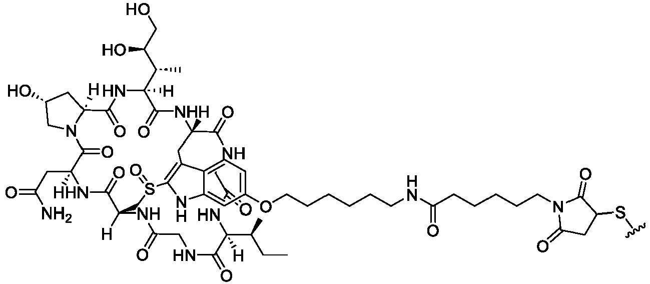

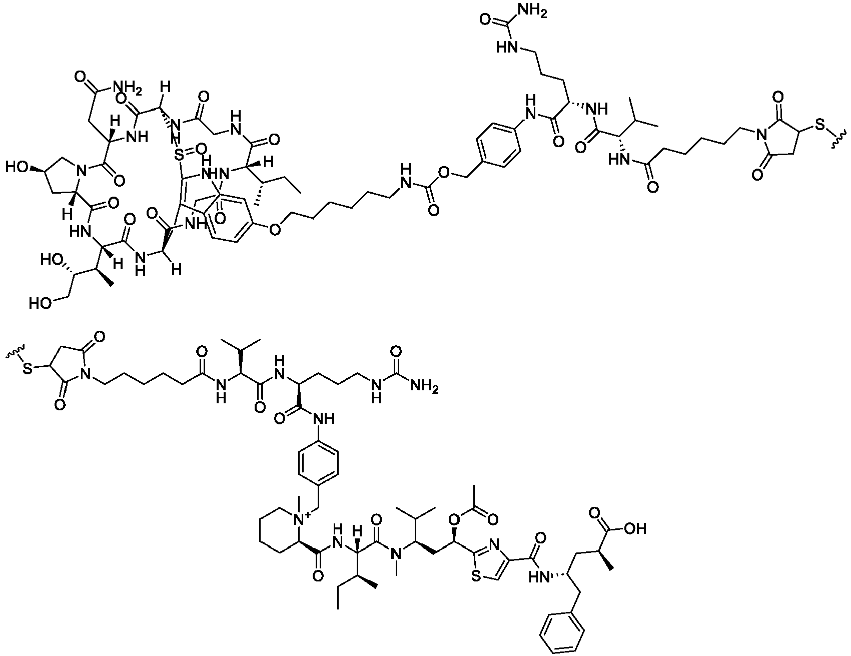

11. The process of preparation of the protein conjugate of formula (II) according to anyone of claims 8 to 10, said process comprising :
The step of reacting a protein conjugate of formula (I) :
Protein'-(L1)ₘ-SH (I)
With a compound of formula (III):
R"-L2-Payload (III)
Where L2 and Payload are defined as in anyone of claims 8 to 10, and R" is a thiol-reactive group, such as maleimide, 3-arylpropiolonitrile, bromo-maleimide, chloroacetamide, bromoacetamide, iodoacetamide, acrylamide, vinyl sulfone, pyridyl disulfide, alpha-halo carbonyl, or vinyl pyridine.

12. The process according to claim 11 comprising preparing the protein conjugate of formula (I) by reacting a mixture of conjugates of formula (IV) :
Protein'-Hal (IV)
With a dithiol compound , a hydrosulfide salt or sulfide salt, where Protein' is defined as in Formula (I) and Hal is a halogen atom chosen from F, Cl, I and Br, said conjugate of formula (IV) being prepared by reducing and re-bridging the sulfur atoms of Protein, said reducing and re-bridging comprising :
Reacting Protein with a reducing agent and with a re-bridging agent of formula (B): 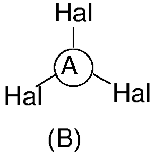
where Protein is a natural or synthetic protein or fragment thereof;
A is defined as in anyone of claims 1 to 7; and
Hal is a halogen atom chosen from F, Cl, I and Br.

13. The compound of formula (B-1): 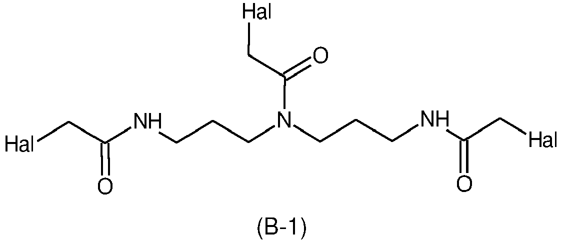 Where Hal is a halogen atom.

14. A pharmaceutical composition comprising a conjugate of formula (II) or mixture thereof according to anyone of claims 8 to 10.

15. The conjugate of formula (II) or the mixture thereof as defined in anyone of claims 8 to 10 for use for treating and/ diagnosing a condition or disorder chosen from the group consisting in tumor, cancer, autoimmune disease, or infectious disease in a subject.
